(19) Europäisches Patentamt / European Patent Office / Office européen des brevets

(11) **EP 3 132 269 B1**

(12) **EUROPEAN PATENT SPECIFICATION**

(45) Date of publication and mention of the grant of the patent:
**25.12.2019 Bulletin 2019/52**

(21) Application number: **15714830.5**

(22) Date of filing: **09.04.2015**

(51) Int Cl.:
*G01N 33/68* (2006.01)    *C07K 16/18* (2006.01)

(86) International application number:
**PCT/EP2015/057761**

(87) International publication number:
**WO 2015/158608 (22.10.2015 Gazette 2015/42)**

(54) **DIAGNOSIS OF CHRONIC KIDNEY DISEASE BY QUANTITATIVE ANALYSIS OF POST-TRANSLATIONAL MODIFICATIONS OF PLASMA PROTEINS**

DIAGNOSE VON CHRONISCHER NIERENERKRANKUNG DURCH QUANTITATIVE ANALYSE VON POSTTRANSLATIONALEN MODIFIKATIONEN VON PLASMAPROTEINEN

DIAGNOSTIC D'UNE NÉPHROPATHIE CHRONIQUE PAR ANALYSE QUANTITATIVE DE MODIFICATIONS POST-TRANSLATIONNELLES DES PROTÉINES PLASMATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.04.2014 EP 14165189**

(43) Date of publication of application:
**22.02.2017 Bulletin 2017/08**

(73) Proprietor: **eXcorLab GmbH**
**63784 Obernburg (DE)**

(72) Inventors:
• **LEMKE, Horst-Dieter**
**63785 Obernburg (DE)**
• **RÜTH, Marieke**
**63785 Obernburg (DE)**
• **JANKOWSKI, Joachim**
**52074 Aachen (DE)**
• **JANKOWSKI, Vera**
**52074 Aachen (DE)**

(74) Representative: **Grünecker Patent- und Rechtsanwälte**
**PartG mbB**
**Leopoldstraße 4**
**80802 München (DE)**

(56) References cited:
• **LUIZ CARLOS CICHOTA ET AL: "Evaluation of ischemia-modified albumin in anemia associated to chronic kidney disease", JOURNAL OF CLINICAL LABORATORY ANALYSIS, vol. 22, no. 1, 1 January 2008 (2008-01-01), pages 1-5, XP055145337, ISSN: 0887-8013, DOI: 10.1002/jcla.20226**
• **CARLO DONADIO ET AL: "Albumin modification and fragmentation in renal disease", CLINICA CHIMICA ACTA, ELSEVIER BV, AMSTERDAM, NL, vol. 413, no. 3, 9 November 2011 (2011-11-09), pages 391-395, XP028346603, ISSN: 0009-8981, DOI: 10.1016/J.CCA.2011.11.009 [retrieved on 2011-11-19]**
• **AGARWAL R: "On the nature of proteinuria with acute renal injury in patients with chronic kidney disease", AMERICAN JOURNAL OF PHYSIOLOGY - RENAL PHYSIOLOGY 200502 US, vol. 288, no. 2 57-2, February 2005 (2005-02), pages F265-F271, XP002730893, ISSN: 0363-6127**
• **E. O. APOSTOLOV: "Quantification of Carbamylated LDL in Human Sera by a New Sandwich ELISA", CLINICAL CHEMISTRY, vol. 51, no. 4, 3 February 2005 (2005-02-03), pages 719-728, XP055156536, ISSN: 0009-9147, DOI: 10.1373/clinchem.2004.044032**

- EUGENE O. APOSTOLOV ET AL: "Carbamylated Low-Density Lipoprotein: Nontraditional Risk Factor for Cardiovascular Events in Patients With Chronic Kidney Disease", JOURNAL OF RENAL NUTRITION, vol. 22, no. 1, 1 January 2012 (2012-01-01), pages 134-138, XP055156542, Pubmed ISSN: 1051-2276, DOI: 10.1053/j.jrn.2011.10.023
- CHEN HUANG-HAN ET AL: "Detection of total and A1c-glycosylated hemoglobin in human whole blood using sandwich immunoassays on polydimethylsiloxane-based antibody microarrays.", ANALYTICAL CHEMISTRY 16 OCT 2012, vol. 84, no. 20, 16 October 2012 (2012-10-16), pages 8635-8641, XP002733322, ISSN: 1520-6882
- HIGASHIMOTO Y ET AL: "Human p53 is phosphorylated on serines 6 and 9 in response to DNA damage-inducing agents", JOURNAL OF BIOLOGICAL CHEMISTRY, AMERICAN SOCIETY FOR BIOCHEMISTRY AND MOLECULAR BIOLOGY, US, vol. 275, no. 30, 28 July 2000 (2000-07-28), pages 23199-23203, XP002303535, ISSN: 0021-9258, DOI: 10.1074/JBC.M002674200
- SMITH W G J ET AL: "Carbamylated haemoglobin in chronic renal failure", CLINICA CHIMICA ACTA, ELSEVIER BV, AMSTERDAM, NL, vol. 178, no. 3, 30 December 1988 (1988-12-30), pages 297-303, XP023399657, ISSN: 0009-8981, DOI: 10.1016/0009-8981(88)90238-0 [retrieved on 1988-12-30]
- MEANS G E ET AL: "CHEMICAL MODIFICATIONS OF PROTEINS: HISTORY AND APPLICATIONS", BIOCONJUGATE CHEMISTRY, AMERICAN CHEMICAL SOCIETY, US, vol. 1, no. 1, 1 January 1990 (1990-01-01), pages 2-12, XP000236570, ISSN: 1043-1802, DOI: 10.1021/BC00001A001
- Joe T Kimmel: "Guanidination of Proteins" In: "Methods in Enzymology", 31 December 1967 (1967-12-31), Elsevier, XP055482517, pages 584-589, DOI: https://doi.org/10.1016/S0076-6879(67)11072-0, * table 1 *

## Description

### Field of the Invention

The present invention relates to methods for diagnosing and/or monitoring the onset or progression of early stage chronic kidney disease (CKD) in a patient by the analysis of post-translational modification of plasma proteins according to the claims.

The present invention provides improved means for early diagnosing chronic kidney disease (CKD) especially for the early recognition of CKD according to the claims.

### Introduction

At present there is a dramatic increase in the prevalence and incidence of chronic kidney disease (CKD) [Plantinga LC. "Socio-economic impact in CKD". Nephrol Ther. 2013; Hallan S.I., et al. JAMA. 2012;308:2349-2360]. The present diagnostic tools for diagnosing CKD rely on the measurement of creatinine concentration in the blood. Plasma creatinine is an important indicator of renal health because it is an easily-measured by-product of muscle metabolism that is excreted unchanged by the kidneys. Therefore, creatinine levels in blood and urine may be used to calculate the creatinine clearance (CrCl), which correlates with the glomerular filtration rate (GFR). Blood creatinine levels are also used alone to calculate the estimated GFR (eGFR).

The GFR can be calculated on the basis of several equations, e.g. the MDRD formula, which includes creatinine concentration, age, gender and the ethnic origin of the patient, or the Cockroft-Gault-formula, which includes creatinine concentration, age, gender and body weight.

A further method for determining the GFR is based on the measurement of cystatin C.

The different methods for evaluating or assessing the GFR yield different results regarding the diagnosis of the CKD stages. The accuracy of attributing the correct stage of the CKD, however, is necessary in order to take the adequate therapeutic means in good time. All the known methods have in common that they are limited to measure the renal failure that has already occurred, i.e. they are not suitable to detect the onset of chronic renal failure.

Post-translationally modified proteins have gained increased interest in recent years, since these modifications are assumed to have an strong impact on the genesis and progression of a large number of diseases such as atherosclerosis, diabetes mellitus and chronic kidney disease (CKD). Post-translational modifications alter physiological and pathophysiological properties of proteins. Some post-translational modifications have been described in recent years, for example advanced glycosylation [Sun Y.M. et al. Biochem Biophys Res Commun. 2013;433:359-361], oxidation of plasma proteins [Prakash M et al., Indian J Nephrol. 2010;20:9-14], nitrosylation [Zager R.A et al., Am J Physiol Renal Physiol. 2006;291:F546-556], carbamylation [Wang, 2007, Nature Medicine 13, pp. 1176-84] or acetylation [Gaikwad A.B. et al., Am J Pathol. 2010;176:1079-1083].

Han et al. (Am. J. Kidney. Dis. (1997) 30, pp. 36-40) found a progression of levels of carbamylated haemoglobin (CarHb) during uremia and that CarHb measurements may be used to differentiate acute renal failure (ARF) from chronic renal failure (CRF), however the methods of Han *et al.* do not allow to concretely attribute a diagnosis to a given level of CarHb observed. CarHb may therefore be indicative, but not sufficient to diagnose ARF or CRF. A single measurement of the CarHb level is also considered not useful as an indicator of the adequacy of dialysis (Kairaitis et al. (Nephrol. Dial. Transplant (2000) 15, pp. 1431-7).

Apostolov et al. (JASN (2010) 21, pp. 1852-7) reported that carbamylated LDL (cLDL) is a risk factor for uremia-induced atherosclerosis, and that chronic uremia stimulated LDL carbamylation. cLDL has also be reported to be a risk factor for developing cardiovascular events in patients with CKD (Apostolov et al., J. Renal. Nutr. (2012) 22, pp. 134-8).

Carbamylated haemoglobin has been suggested as a posttranslationally modified protein marker for discriminating between acute and chronic renal failure. Carbamylated LDL (cLDL) was equally proposed to represent a useful marker for CKD and an sandwich ELISA assay has been developed to determine plasma cLDL. Said assay allows to safely discriminate healthy individuals from patients suffering from end stage renal disease (Apostolov *et al., supra* and Apostolov, E.O., Clinical Chemistry 51:719-28 (2005)). An influence of carbamylated LDL on the onset and progression of various diseases due to the oxidative character of cLDL was postulated (Apostolov, E.O., et al., J. Renal Nutr. 22:134-8 (2012)).

Luiz Carlos Cichota et al. (Journal of Clinical Laboratory Analysis 22:1-5 (2008)) detected ischemia-modified albumin in patients suffering from CKD associated anemia. Carlo Donadio et al. (Clinica Chimica Acta 413:391-395 (2012)) discloses albumin fragmentation in patients suffering from end stage renal disease. Agarwal, R. (Am. J. Physiol. 288:F265-71 (2005)) discloses the occurrence of carbonylated proteins in urine of CKD patients. Chen Huang-Han et al. (Analyt. Chem. 84:8635-41 (2012)) discloses antibodies to A1c-glycosylated hemoglobin and their use in the diagnosis of diabetes. Phosphoserine-specific antibodies were used in the context of p53 by Higashimoto Y. et al. (JBC 275 (2000), p. 23199-23203). Smith W.G. et al. (Clin. Chim. Acta 178 (1988), p. 297-303) relates to a gas chromatographic detection of a secondary metabolite of a PTM of hemoglobin.

Hence, there exist several reports on how chronic renal failure or chronic uremia affects plasma protein components posttranslationally. These observations allow a correlation with chronic renal diseases or an attribution as risk factors for developing further secondary diseases on the background of an existing chronic renal failure, but they do not allow or suggest an early diagnosis of chronic kidney disease or of an impairment of renal function prior to the development of chronic renal conditions. Furthermore, the existing methods do not allow to securely attribute a diagnosis on the basis of a single measurement.

There is a need in the art for diagnostic markers for early stages of renal impairment or chronic kidney disease. There is a need in th art for markers that allow a reliable diagnosis of CKD on the basis of a single measurement.

Methods available for the precise assessment of post-translational modifications which are applicable for a large number of clinical samples and/or the clinical routine presently are also limited. The current methods are limited by only diagnosing impaired kidney function based on physiological parameters, i.e. they are limited to stages of the disease where actual organ damage has already occurred.

Hence, there remains a need for methods that adequately and precisely allow the diagnosis of CKD, in particular early and very early stages of CKD and that allow a diagnosis of CKD prior to the onset of a concrete physiological impact on the kidney function, i.e. before the GFR is impaired at all.

The present invention fulfils this need by providing methods that are based on the identification and quantification of post-translational modification of plasma proteins and their correlation with very early stages of CKD as detailed in the claims.

## Summary of the Invention

In a first aspect the present disclosure relates to a method for diagnosing and/or monitoring chronic kidney disease (CKD) in an individual, the method comprising the steps of (a) analyzing the pattern of posttranslational modifications of a plasma protein of an individual subject to be diagnosed as outlined in the appended set of claims, (b) comparing the posttranslational modification pattern of the plasma protein of the individual to be diagnosed with the posttranslational modification pattern of the plasma protein of a healthy individual (i.e. an individual not suffering from CKD); and (c) detecting a difference in the posttranslational modification pattern of the plasma protein of the individual to be diagnosed as compared to the posttranslational modification pattern of the plasma protein from the healthy individual and diagnosing CKD, if the plasma protein of the individual to be diagnosed exhibits at least one posttranslational modification according to a) above that is absent in the plasma protein of the individual not suffering from CKD. The analysis of the posttranslational modification pattern is performed *in vitro.* The analysis of the posttranslational modification pattern is performed on an isolated sample of the individual; preferably the sample is a blood or plasma sample that has been obtained from the individual prior to the analysis. The analysis may well be performed in the absence of the individual to be diagnosed. Where the analysis of the post translational modification pattern of the plasma protein involves a carbamylation or oxidation, the plasma protein is not LDL or haemoglobin.

The invention, as outlined above, relates to diagnosing CKD as defined in the claims, if the plasma protein as defined in the claims of the individual to be diagnosed exhibits at least one posttranslational modification as defined in the claims that is absent in the plasma protein of the healthy individual (not suffering from CKD). A skilled person would understand that naturally also healthy individuals may exhibit a very low level of posttranslational modifications on plasma proteins. For example approximately 10% of the albumin in normal human serum is modified by non-enzymatic glycosylation, primarily at the epsilon-amino group of lysine residue 525 [Shaklai N. et al., J. Biol. Chem. 259:3812-3817(1984)]. Diagnosing CKD can therefore also be made by detecting an elevated level of posttranslational modifications of one or more plasma proteins; the elevated level of posttranslational modification can be confined to a specific site of the one or more plasma proteins, or more than one specific site of the respective plasma protein(s). Preferably, the diagnosis of CKD is made by detecting one or more posttranslational modifications on sites of a plasma protein that are not modified in a healthy individual. Hence, the diagnosis can be made by determining the total amount of posttranslational modifications of a specific plasma protein and comparing that total amount to the total amount of that same type of post translational modification of the same plasma protein from a healthy control. A diagnosis can therefore be made on the basis of detecting a relative increase of posttranslational modifications of a selected plasma protein species. Hence, the diagnosis can be made by detecting at least one posttranslational modification in a selected plasma protein that does not occur in a healthy individual. For posttranslational modification on plasma proteins that are known to have a defined base level of modification the diagnosis can be made if the relative increase of that posttranslational modification on that plasma protein species is detectable within a signal-to-noise ratio of 3 or more. Preferably, CKD is diagnosed if an increase in the posttranslational modification level of one or more selected post translational modification(s) of a plasma protein species is detectable. Preferably the increase is at least 1,05 fold or higher, at least 1,1 fold or higher or at least 1,2 fold or higher. The increase can be at least 1,5 fold higher, preferably at least 2 fold, more preferably at least 3 fold, or higher than 6 fold as compared to the corresponding site of the same plasma protein obtained from a healthy individual.

Depending on the base level of posttranslational modification of the plasma protein, there is no upper limit for the relative increase in the level of posttranslational modification, hence the level may be increased by the factor of 10, 100, 1000, etc.. Preferably, the diagnosis can be made on the basis of a single determination of the posttranslational modification status of the selected plasma protein or the total amount of modifications in the total protein content obtained from the individual to be diagnosed. That is, the posttranslational modification of the plasma protein is unique to individuals having CKD, or being at risk of developing CKD.

In an alternative method, the total amount of modifications or the total amount of a specific type of posttranslational modification (e.g. guanidylation) in the total plasma protein content is determined and compared to the total amount of modifications, or modifications of a specific type (e.g. guanidylation) of the total plasma protein content of a healthy individual. This method does not require to purify a single plasma protein species and can be performed on whole serum samples.

The condition diagnosed with the inventive method is chronic kidney disease in an early stage, that is the CDK diagnosed is early stage CKD. The inventive methods for diagnosing CKD (or a precursor condition, i.e. a condition leading to CKD) can diagnose the condition in an individual that does not yet exhibit an elevated level of creatinine and does not exhibit a clinically detectable or clinically relevant stage of albuminurea. Preferably, the individual to be diagnosed in stage 0 (see Table 2) exhibits a glomerular filtration rate (GFR) of >90mL/min and an albuminurea of less than 10 mg/g, preferably less than 5 mg/ml and more preferably less than 1 mg/ml, most preferably the individual does not yet exhibit any measurable albuminurea.

In the above methods for diagnosing CKD, the plasma protein is preferably selected from the group consisting of albumin (P02768 (UniProt, Database version 200), e.g. SEQ ID NO:1 or 2), beta-2 microglobulin ($\beta$2MG) (P61769 (UniProt, Database version 121), e.g. SEQ ID NO:3), cystatin C (P01034 (UniProt, Database version 165), e.g. SEQ ID NO:4), transferrin (P02787 (UniProt, Database version 178), e.g. SEQ ID NO:5), and retinol binding protein (P02753 (UniProt, Database version 166), e.g. SEQ ID NO:6). The present disclosure also encompasses any cleavage products that have been reported and that are specified in the above UniProt Database entries such as e.g. for retinol-binding protein 4 it is known that it is cleaved into the following 4 chains: Plasma retinol-binding protein(1-182), Plasma retinol-binding protein(1-181), Plasma retinol-binding protein(1-179), and Plasma retinol-binding protein(1-176). The analysis may involve the detection of the posttranslational modification of only one serum protein, or it may involve the detection of the posttranslational modification pattern of two, three, four, or more of these plasma proteins. In a particularly preferred embodiment, the plasma protein is human serum albumin, preferably human serum albumin of SEQ ID NO: 1 or 2. A skilled person will understand that natural variants, isoforms and/or point mutations of the above-listed plasma proteins may exist. The present invention is also applicable to these naturally occurring variants plasma proteins such as the isoform 1 and 2 of human serum albumin (SEQ ID NOs 1 and 2 respectively).

The posttranslational modification that is subject of analysis is preferably selected from the group consisting of: guanidylation (also often referred to as guanidination or as guanidinylation), monoglycosylation, diglycosylation such as 2xHex (addition of a glucose molecule to an amino acid and subsequent addition glucose molecule to the first glucose molecule), formylation, nitrosylation, carbamylation, acetylation, carbamidomethylation, methylation, dimethylation, citrullination and carboxymethylation. Oxidation is a rather unspecific posttranslational modification and due to its nature of occurring non-specifically, it is not regarded as a useful posttranslational modification for the sake of the present invention unless specifically indicated for a selected plasma protein. Particularly preferred posttranslational modifications are selected from the group consisting of guanidylation of albumin (e.g. human serum albumin), the guanidylation of $\beta$2MG (preferably on at least one lysine residue), the oxidation of $\beta$2MG (preferably, on at least one methionine residue), the formylation of $\beta$2MG (preferably on the N-terminus of $\beta$2MG), the carbamidomethylation of $\beta$2MG (preferably on at least one cysteine residue), the carbamylation of $\beta$2MG (preferably on at least one lysine residue), the carboxymethylation of $\beta$2MG (preferably on at least one cysteine residue), the formylation of cystatin C (preferably on the N-terminus), the carboxymethylation of cystatin C (preferably on at least one cysteine residue), the carbamylation of cystatin C (preferably on at least one lysine residue), the guanidylation of transferrin (preferably on at least one lysine residue), and the formylation of retinol binding protein (preferably on the N-terminus). As outlined above, each one of the aforementioned posttranslational modifications can be used alone, or in combination with each other in order to diagnose CDK. It is preferred to monitor one, two, three, four, five, or six (as far as applicable to the selected plasma protein) posttranslational modifications of one selected plasma protein, or to monitor a specific posttranslational modification (e.g. lysine guanidylation) of more than one plasma protein (e.g. of both albumin and transferrin), or of total protein in plasma. Where the posttranslational modification is a carbamylation, the plasma protein is not LDL or haemoglobin.

Where the posttranslational modification is a guanidylation of human serum protein, the position of the posttranslational modification is occurring on K36, K44, K183, K186, K198, K205, K219, K300, K375, K383, K463, K468, K499, K548, K560, K562, K565, K569, K581, K584, K588, K597 and/or K598 of human serum albumin of SEQ ID NO:1, or the corresponding position of a naturally occurring variant of human serum albumin. Where the posttranslational modification is the guanidylation of $\beta$2MG at a lysine residue, the posttranslational modification preferably occurs at position K26, K66, K68, K114, where the posttranslational modification is the oxidation of $\beta$2MG on at least one methionine residue, the posttranslational modification occurs at position M119, or on the corresponding residue(s) of a naturally occurring variant of any of the aforementioned proteins.

In the above methods for diagnosing CKD any suitable method for detecting the posttranslational modification of the plasma protein may be used. Exemplary methods for detecting the difference in the posttranslational modification pattern of the plasma protein are mass spectrum analysis of the isolated plasma protein, or immunological methods based on antibodies that specifically discriminate between a post-translationally modified protein and a non-post-translationally modified protein. These methods include ELISA and Western Blot. A further method for detecting posttranslational modifications of proteins is 2D page followed by colorimetric detection of the post-translational modification.

Where the detection of the posttranslational modification of the plasma protein is performed by mass spectrum analysis, the technique used is preferably MALDI-TOF-TOF. Particularly preferred is a MALDI-TOF-TOF-MS/MS analysis. As it is apparent, specific modifications such as glycosylation may also be observed on peptide fragments from the respective plasma protein, only requiring a MALDI-TOF analysis. Any such peptide fragments that carry a specific posttranslational modification can be used as a marker in the diagnostic methods of the present invention. In any of the above methods, due to the specific physical characteristics of these marker peptides, these marker peptides can be detected in samples that comprise, consist of or consist essentially of the selected plasma protein, or that comprise, consist of or consist essentially of a plasma sample obtained from the individual to be diagnosed. This allows for a cost-effective straight forward analysis of samples, requiring only a moderate level of instrumentation.

In another aspect, the present invention relates to the *in vitro* use of an antibody according to the claims that specifically binds to the post-translationally modified plasma protein, wherein said antibody does not specifically bind to the plasma protein which is not post-translationally modified, i.e. the antibody binds to a plasma protein of an individual afflicted with CKD or exhibiting a condition that may develop into CKD, while the antibody does not bind to the same plasma protein obtained from a healthy individual (not suffering from CKD, and/or not being susceptible of developing CKD). Suitably, the $K_D$ of the antibody with respect to the plasma protein that is not post-translationally modified is $5x10^{-5}$ or higher. In a preferred embodiment the post-translationally modified plasma protein is selected from the group consisting of albumin (P02768 (UniProt, Database version 200), e.g. SEQ ID NO:1 or 2), beta-2 microglobulin ($\beta$2MG) (P61769 (UniProt, Database version 121), e.g. SEQ ID NO:3), cystatin C (P01034 (UniProt, Database version 165), e.g. SEQ ID NO:4), transferrin (P02787 (UniProt, Database version 178), e.g. SEQ ID NO:5), and retinol binding protein (P02753 (UniProt, Database version 166), e.g. SEQ ID NO:6). Particularly suitable is an antibody that specifically binds to guadinylated human serum albumin (i.e. the antibody binds with a higher affinity to guadinylated human serum albumin as compared to non-guadinylated human serum protein).

Further disclosed is a diagnostic kit comprising the above described antibody. The kit may further contain secondary antibody, as well as positive and negative controls and further means for detection (like, e.g. chemiluminescence detection reagents). Where the plasma protein to be detected is human serum albumin, the positive control is, e.g. *in vitro* guadinylated human serum protein, or human serum protein obtained from a patient being previously diagnosed positively for CKD. The negative control may be recombinantly produced human serum protein from a source that does not perform or exhibit the posttranslational modification of the respective protein or human serum protein obtained from a healthy individual. The diagnostic kit may contain instructions for use of the kit's ingredients in order to perform the diagnostic methods of the present invention.

The described antibody may be used in any of the above methods for diagnosing CKD.

## Description of the Figures

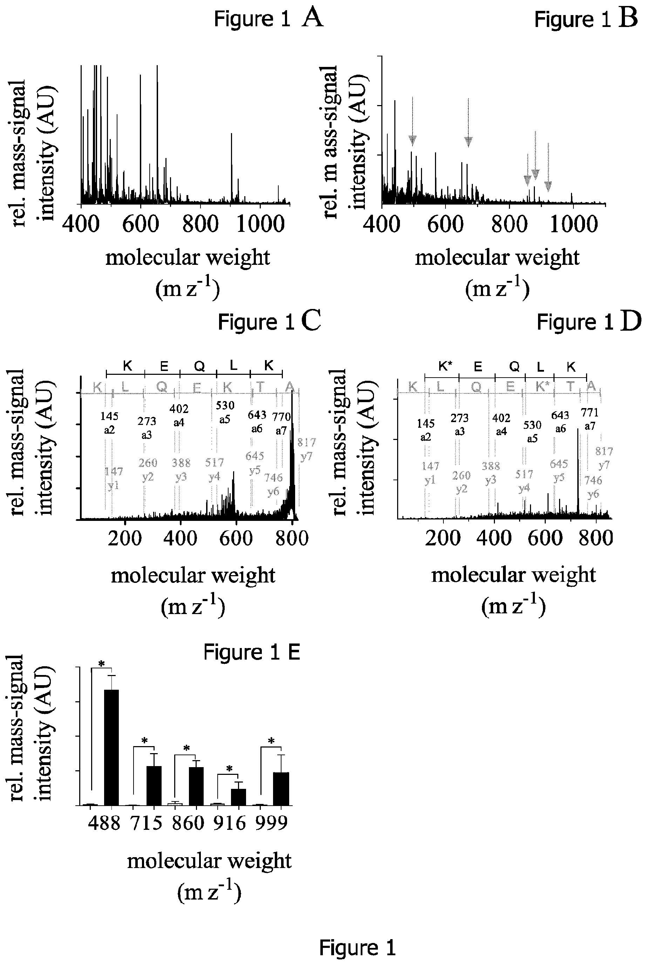

**Figure 1:** (A) Characteristic fingerprint-spectra of albumin after tryptic digestion isolated from plasma of healthy control subjects. (B) Characteristic fingerprint-spectra of albumin after tryptic digestion isolated from CKD patients. Grey arrows indicate significant differences to the mass-signals shown in **Figure 1A.** (C) Characteristic MS/MS-mass spectrum of the mass-signal at 817.19 Da of albumin after tryptic digestion isolated from the plasma of healthy control subjects. (D) Characteristic MS/MS-mass spectrum of the mass-signal at 858.54 Da of albumin after tryptic digestion isolated from CKD patients. (E) Quantification of lysine guanidylation in CKD patients (black bars) compared to unmodified lysines in healthy control subjects (grey bars).

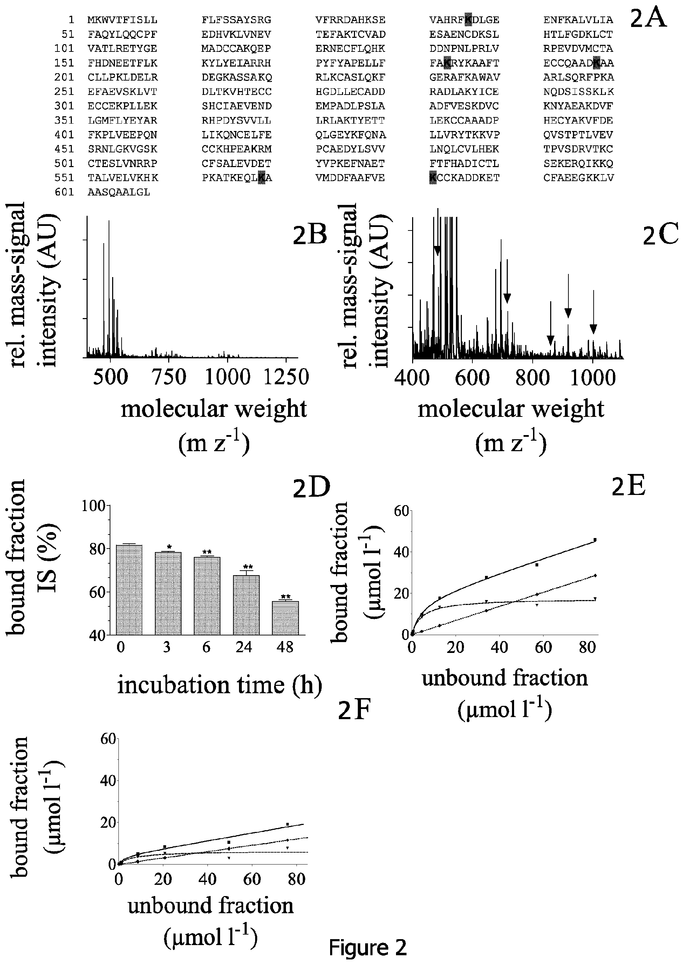

**Figure 2:** (A) Amino acid sequence of albumin. The guanidylated lysine groups indicated by red boxes were detected in both in modified albumin isolated from CKD patients and in in-vitro guanidylated albumin with impaired binding capacity. (B) Characteristic mass spectrum of commercially available albumin before *in-vitro* guanidylation. (C) Characteristic mass spectrum of commercial available albumin after *in-vitro* guanidylation. Arrows indicate significant differences of the mass-spectrum compared to the mass-spectrum shown in **Figure 2B.** (D) Effect of increased *in-vitro* guanidylation incubation time on binding of commercial albumin for indoxyl sulfate (* $p < 0.01$; ** $p < 0.005$).

(E) Total (■), specific (▼) and unspecific (♦) binding of commercial available albumin before in-vitro guanidylation.
(F) Total (■), specific (▼) and unspecific (♦) binding of commercial available albumin after *in-vitro* guanidylation.

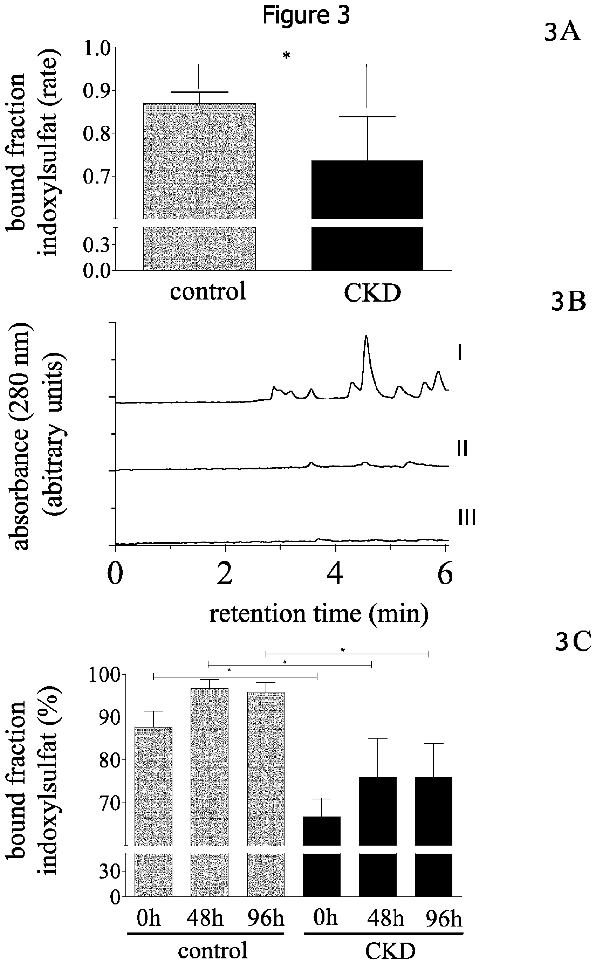

**Figure 3:** (A) Binding capacity of albumin isolated from healthy control subjects (grey bar) and CKD patients (black bar, p < 0.05). (B) Characteristic reversed-phase chromatography of albumin isolated from CKD patients before (I) and after (II) size-exclusion chromatography, and then dialysis against 1M NaCl (III). (C) Effect of increased dialysis time on protein bound fraction of albumin isolated from healthy control subjects (grey bar) and CKD patients (black bar, p < 0.05).

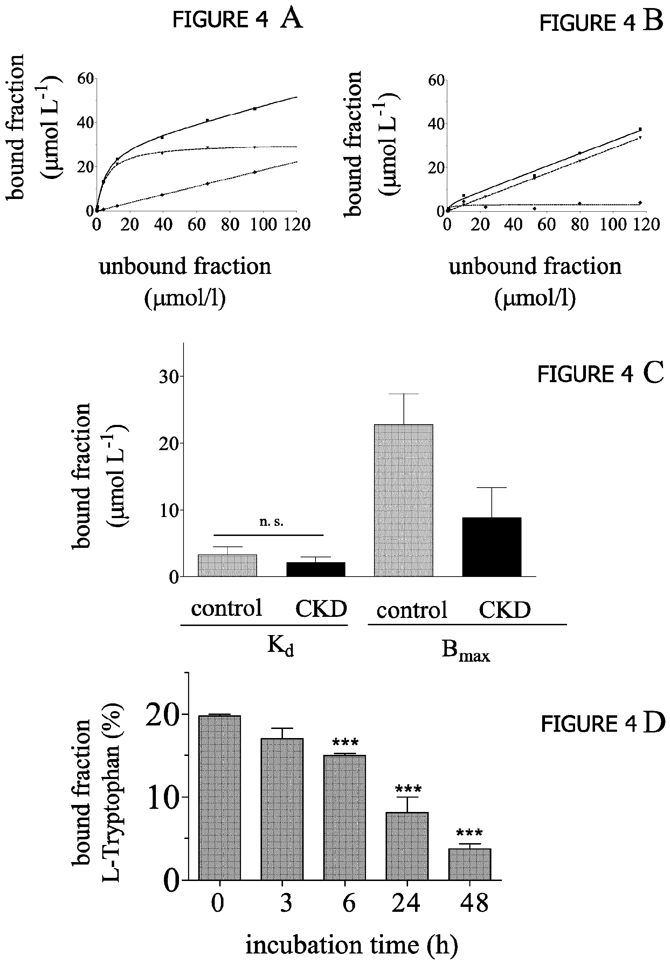

**Figure 4:** (A) Total (■), specific (▼) and unspecific (♦) binding of albumin isolated from healthy control subjects. (B) Total (■), specific (▼) and unspecific (♦) binding of albumin isolated from CKD patients. (C) Dissociation constant ($K_d$) and number of specific binding sites ($B_{max}$) of albumin isolated from healthy control subjects (grey bars) and CKD patients (black bars). (D) Effect of increased *in-vitro* guanidylation incubation time on protein bound fraction of albumin for L-tryptophan (* p< 0.01; ** p< 0.005).

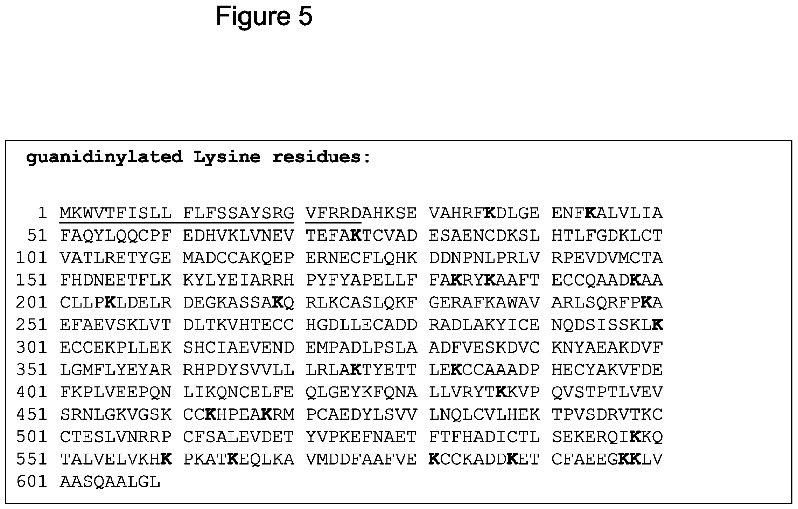

**Figure 5:** Figure 5 denotes the sequence of human serum albumin of SEQ ID NO:1, where **boldface** indicates a position where a lysine guanidylation was found in CKD (stage 5) patients.

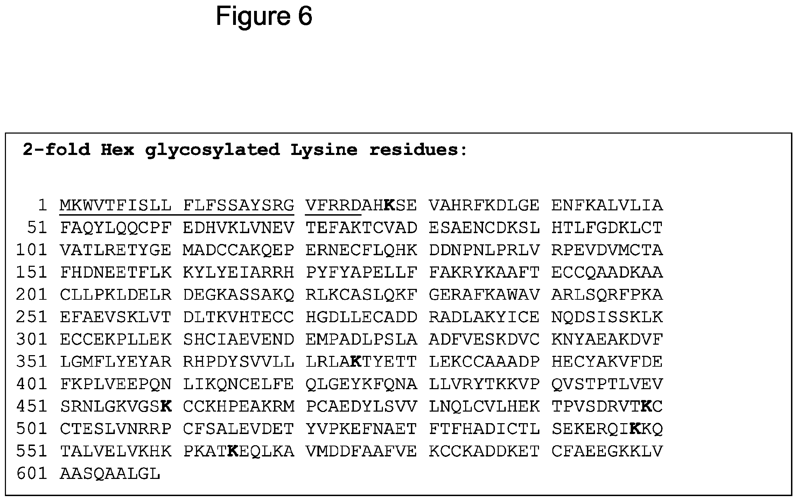

**Figure 6:** Figure 5 denotes the sequence of human serum albumin of SEQ ID NO:1, where **boldface** indicates a position where a 2xglycosylation (2xHex) was found in CKD (stage 5) patients.

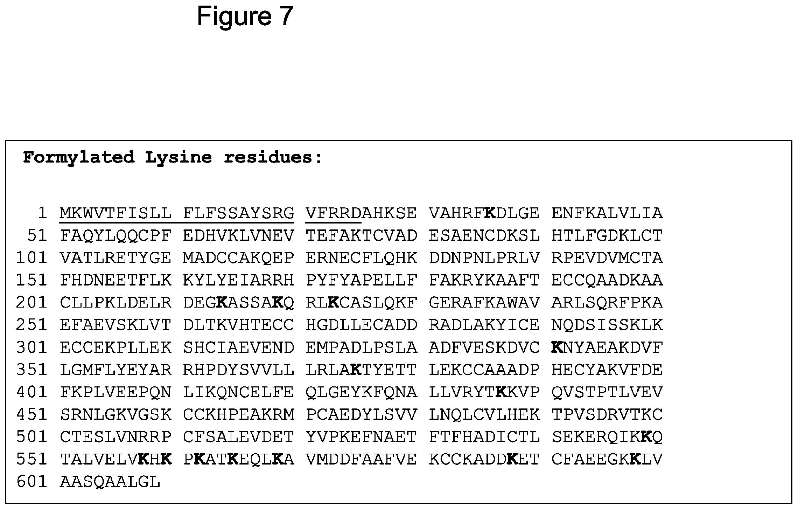

**Figure 7:** Figure 5 denotes the sequence of human serum albumin of SEQ ID NO:1, where **boldface** indicates a position where a formylated lysine was found in CKD (stage 5) patients.

## Detailed Description of the Invention

The present invention is based on the finding that posttranslational modifications of plasma proteins are indicative for the onset and the progression of chronic kidney disease (CKD). Since plasma proteins are an easily accessible source of proteins which can be obtained by routine methods, they are particularly suited for diagnosis. The finding that posttranslational modifications are reliable diagnostic markers for (early) CDK enables for the first time to make a reliable diagnosis on the molecular level. The diagnosis on the basis of the analysis of the posttranslational modification pattern of plasma proteins allows making a diagnosis on the basis of a single measurement. The methods of the present invention are also not impaired by other environmental circumstances that may impact the biomarkers typically used to asses CKD like the glomerular filtration rate or albuminurea, that are known to vary over the course of the day (see e.g. Hansen, H.P. et al., Kidney International (2002) 61, 163-168).

One major obstacle in understanding the effects of post-translational modified proteins and peptides has been the difficulty in quantifying the post-translational modification of e.g. plasma proteins. Up to now there is no method available for the quantification of specific post-translational modification of plasma proteins like albumin in a large number of samples. The presently available methods do also not allow for a precise quantitative analysis of post-translational modifications of clinically relevant plasma proteins.

The present disclosure for the first time provides means for quantitatively assaying post-translational modifications and allows to identify and define threshold values for specific post-translational modifications for specific stages of chronic kidney disease.

The term "diagnosis" relates to the finding of a pathological condition in an individual. It also relates to the finding of a condition that is non-pathological, but that does not occur in a healthy individual, which non-pathological condition may, if left untreated, develop into a pathological condition.

The term "individual" means for the sake of the present invention a human subject.

The term "posttranslational modification" refers to a covalent chemical alteration of a polypeptide or peptide amino acid sequence at any one of the peptide backbone residues, the amino acid side chains or the N- or C-terminus. Posttranslational modifications involving addition of smaller chemical groups comprise acylation, e.g. O-acylation (esters), N-acylation (amides), S-acylation (thioesters), acetylation (the addition of an acetyl group, either at the N-terminus of the protein or at lysine residues), formylation, alkylation (the addition of an alkyl group, e.g. methyl, ethyl; e.g. methylation is the addition of a methyl group, usually at lysine or arginine residues), amide bond formation, amidation at C-terminus, amino acid addition such as arginylation, a tRNA-mediation addition, polyglutamylation (covalent linkage of glutamic acid residues to the N-terminus),polyglycylation, (covalent linkage of one to more than 40 glycine residues), butyrylation, gamma-carboxylation, glycosylation (addition of a glycosyl group to either arginine, asparagine, cysteine, hydroxylysine, serine, threonine, tyrosine, or tryptophan resulting in a glycoprotein) polysialylation (addition of polysialic acid, PSA), malonylation, hydroxylation, iodination, nucleotide addition such as ADP-ribosylation, oxidation, phosphate ester (O-linked) or phosphoramidate (N-linked) formation, phosphorylation (the addition of a phosphate group, usually to serine, threonine, and tyrosine (O-linked), or histidine (N-linked)), adenylylation (the addition of an adenylyl moiety, usually to tyrosine (O-linked), or histidine and lysine (N-linked)), propionylation, pyroglutamate formation, addition of S-glutathionylation, S-nitrosylation, succinylation (addition of a succinyl group to lysine), sulfation (addition of a sulfate group to a tyrosine), and selenoylation (co-translational incorporation of selenium in selenoproteins). Further posttranslational modifications involving non-enzymatic additions *in vivo* such as glycation (the addition of a sugar molecule to a protein without the controlling action of an enzyme), and other posttranslational modifications that involve changing the chemical nature of amino acids, such as citrullination, or deimination (the conversion of arginine to citrulline), deamidation (the conversion of glutamine to glutamic acid or asparagine to aspartic acid), eliminylation (the conversion to an alkene by beta-elimination of phosphothreonine and phosphoserine, or dehydration of threonine and serine, as well as by decarboxylation of cysteine), and carbamylation (the conversion of lysine to homocitrulline). Posttranslational modifications include guanidylation, mono- and di-glycosylation (such as e.g. 2xHex (addition of a glucose molecule to an amino acid and subsequent addition of a glucose molecule to the first glucose molecule), formylation, nitrosylation, carbamylation, acetylation, carbamidomethylation, certain specific types of oxidation, methylation, dimethylation, citrullination and carboxymethylation. As oxidation is a rather unspecific posttranslational modification, only those specific oxidations are within the scope of the present invention that have been found to be related to CKD, such as the oxidation of β2MG on at least one methionine residue. Where the plasma protein is LDL or haemoglobin, the posttranslational modification is not carbamylation or oxidation. Particularly preferred posttranslational modifications are selected from the group consisting of guanidylation of albumin (e.g. human serum albumin), the guanidylation of β2MG (preferably on at least one lysine residue), the oxidation of β2MG (preferably, on at least one methionine residue), the formylation of β2MG (preferably on the N-terminus of β2MG), the carbamidomethylation of β2MG (preferably on at least one cysteine residue), the carbamylation of β2MG (preferably on at least one lysine residue), the carboxymethylation of β2MG (preferably on at least one cysteine residue), the formylation of cystatin C (preferably on the N-terminus), the carboxymethylation of cystatin C (preferably on at least one cysteine residue), the carbamylation of cystatin C (preferably on at least one lysine residue), the guanidylation of transferrin (preferably on at least one lysine residue), and the formylation of retinol binding protein (preferably on the N-terminus) as detailed in the claims.

A given protein, such as a plasma protein may be modified by only one such posttranslational modification or may exhibit several of the same or different posttranslational modifications.

Hence, the "posttranslational modification pattern" used in the present disclosure may refer to only one posttranslational modification or to a plurality of posttranslational modifications of a given protein. Hence, a "difference in posttranslational modification pattern" may be observed if, in comparison with the control, a protein exhibits additional posttranslational modifications, or lacks posttranslational modifications. Furthermore, a difference in the posttranslational modification pattern also encompasses the situation where the posttranslational modification(s) observed in the control has (have) not changed in number or nature, but in the position within the protein species investigated.

The term "detecting" is used in the broadest sense to include both qualitative and quantitative measurements of a target molecule. In one aspect, the detecting method as described herein is used to identify the mere presence of posttranslational modification or a post-translationally modified protein or peptide in sample. In another aspect, the methods are used to test whether the level of posttranslational modifications in a sample is at a detectable level. In yet another aspect, the method can be used to quantify the amount of posttranslational modification of a plasma protein or a post-translationally modified plasma protein or peptide in a sample and further to compare these levels from different samples.

A method of diagnosing involves the detection of at least one posttranslational modification in a particular plasma protein obtained from an individual to be diagnosed, which posttranslational modification is absent in the corresponding particular plasma protein obtained from a healthy individual, or obtained from the corresponding particular plasma protein, produced *in vitro*. As an example, a plasma protein, e.g. human serum albumin, is analyzed for the presence of a guanidylation on the position K36 (of SEQ ID NO:1). Human serum albumin obtained from a healthy individual does not exhibit such a posttranslational modification. If the individual from which human serum albumin was obtained exhibits a guanidylated K36, the individual is diagnosed with CKD, on the basis of the finding of only one posttranslational modification. The present invention also covers methods where the analysis of more than one, e.g. two, three, four, five or six or more posttranslational modifications of a particular plasma protein species is analyzed. These posttranslational modifications of the particular protein may be of the same nature (e.g. guanidylation). For example, the more than one posttranslational modifications analyzed can consist of or comprise one or more of guanidylation of human serum albumin identified in Table 1 below in any combination, e.g. of K36, K174, K188, K569, and/or K581 of human serum albumin (SEQ ID NO:1) in any combination.

The posttranslational modifications of the particular protein may also be of several different natures (e.g. analysis of guanidylation and carbamylation, or of oxidation and formylation, or of guanidylation, carbamidomethylation and oxidation, and so on), as long as the respective plasma protein exhibits these posttranslational modifications on an (early) CDK background.

Synonymously to the above, it is the post translational modification pattern that is analyzed for a particular plasma protein.

The present methods also comprise analyzing at least one type of posttranslational modification on more than one type of plasma protein, e.g. the methods comprise the detection of guanidylated lysine in both human serum albumin and transferrin, or the detection of guanidylated lysine in all of human serum albumin, transferrin and beta-2MG or the posttranslational modification pattern of a sample of whole serum.

Assessing the posttranslational modification level can also be performed with whole plasma samples. In this case, a specific posttranslational modification is determined through detection of the posttranslational modification of the plasma protein by way of an antibody described above, or by digesting the plasma with a protease and subsequently analyzing the sample for the presence of at least one peptide that is specific for a post-translationally modified plasma protein (i.e. having a characteristic mass or mass/charge).

Table 1 below lists some exemplary mass peaks of peptides from a tryptic digest of human serum albumin and their respective shifts that can be observed. The human serum albumin was isolated from hemofiltrates of patients undergoing dialysis. The first number indicating the mass peak of the unmodified peptide (e.g. 1340), the second number indicates the mass peak of the same peptide with the indicated modification (here: 1382, the shift of 1340 to 1382 corresponding to the peptide being modified by guanidylation, i.e. a shift of 42 Da).

*Table 1: Mass peaks of tryptic HSA peptides and corresponding mass shifts after posttranslational modification. H-18, H-04 and H-11 designate the respective identification number of patients (ESRD Class 5HD) undergoing dialysis from which the hemofiltrates were obtained.*

| Probe 2 = Hemofiltrate H-18 | | | Probe 1 Hemofiltrate H-04 | | | Probe 3 Hemofiltrate H-11 | |
|---|---|---|---|---|---|---|---|
| **Guanidinylation** | | | **Guanidinylation** | | | **Guanidinylation** | |
| 1340-1382 | Guanidylation | | 972 - 1014 | Guanidylation | | 972 - 1014 | Guanidylation |
| 463 - 505 | Guanidylation | | 1340 - 1382 | Guanidylation | | 446 -488 | Guanidylation |
| 914 - 998 | 2 Guanidylations | | 874 - 916 | Guanidylation | | 746 -788 | Guanidylation |
| 346 - 388 | Guanidylation | | 914-998 | 2 Guanidylations | | 914 -998 | 2 Guanidylation |
| 672 - 714 | Guanidylation | | 346 - 388 | Guanidylation | | 318 - 360 | Guanidylation |
| 318 - 360 | Guanidylation | | 672 - 714 | Guanidylation | | 808 -850 | Guanidylation |
| | | | 318 - 360 | Guanidylation | | 1295 - 1379 | 2 Guanidylation |
| | | | | | | | |
| **Formylation N-Term** | | | **Formylation N-Term** | | | **Formylation N-Term** | |
| 318 - 346 | Formylation | | 318 - 346 | Formylation | | 318 - 346 | Formylation |
| | | | | | | | |
| **Carbamidomethylation** | | | **Carbamidomethylation** | | | **Carbamidomethylation** | |
| 352 - 409 | Carbamidomethyl | | 352 - 409 | Carbamidomethyl | | 0 | - |
| | | | | | | | |
| **Carbamylation** | | | **Carbamylation** | | | **Carbamylation** | |
| 463 - 506 | Carbamyl | | 463 - 506 | Carbamyl | | 463 - 506 | Carbamyl |
| | | | | | | | |
| **Carboxymethylation** | | | **Carboxymethylation** | | | **Carboxymethylation** | |
| 330 - 388 | Carboxymethyl | | 781 - 839 | 2 Carboxymethyl | | 352 - 468 | Carboxymethyl |
| | | | 330 - 388 | Carboxymethyl | | | |
| | | | 801 - 917 | 2 Carboxymethyl | | | |

It will be understood that the diagnosis can be made on the basis of any combination of the posttranslational modifications that were observed to be specific for (early stages of) CKD, see Table 2 below for a selection of posttranslational modification indicative for (early) CKD.

In a given population of protein molecules, some individual protein molecules may exhibit a certain specific posttranslational modification while other individual protein molecules of the same species do not. In such a case, it is referred to "the extent of posttranslational modification" of a given protein. Synonymously, it is referred to as the "relative amount of posttranslational modification" of the protein. It can be measured by determining the total protein content of the protein species in a sample and subsequently determining the extent of post-translationally modified protein in the total amount of the protein species in the sample. The diagnosis of CKD is made when the extent of posttranslational modification observed in the serum protein obtained from the individual to be diagnosed is significantly detectable, defined as signal/noise (S/N) of >3. For example, the extent of posttranslational modification observed may be at least between 1,05 fold to 100000 fold higher, preferably between 1,1 to 10000 fold higher or 1,5 fold higher, preferably at least 2 fold, more preferably at least 3 fold, 4 fold, 5 fold, 6 fold, or higher than 10 fold higher as compared with the extent of posttranslational modification of the corresponding plasma protein obtained form a healthy control. The extent of posttranslational modification of a plasma protein can also be used to monitor the progression of CKD in an individual, and/or can be used to attribute different stages of CDK as defined by the ICD-10 (see below). An "elevated level" of posttranslational modification of a plasma protein is to be understood as being elevated by at least 1%, 2%, 3%, 4%, 5%, 6%, 7%, 8%, 9%, 10%, 20%, 30%, ..., 100%, 1000% (i.e. 10 fold) or more, as compared to the level of posttranslational modification found in the corresponding protein of a healthy control.

In order to monitor the progression of CKD, e.g. in order to assess whether a given therapeutic measure is effective for reducing the CDK (or the susceptibility to develop CKD) the analysis of the posttranslational modification (pattern) of the plasma protein or the plasma proteins, a sample is obtained from the individual at different points in time, e.g. prior to and then one day and/or one week and/or one month after the onset of a particular therapeutic measure. The presence of the particular posttranslational modification (pattern) or of the particular extent of posttranslational modification that was indicative for making the diagnosis of (early) CKD can then be compared to the posttranslational modification (pattern) or extent of posttranslational modification observed in a sample from a later time point in order to evaluate the effectiveness of the therapeutic measure taken.

A "plasma protein" is a protein that is contained in the serum or plasma of mammalian blood. For the purpose of the present invention it is immaterial whether the plasma protein is a precursor form or a splicing variant, as long as the plasma protein exhibits posttranslational modifications that are specific for an individual suffering from CKD or a condition leading to CKD. Specific plasma proteins that are useful as a marker for CKD comprise albumin (e.g. human serum albumin), β2MG, cystatin C, transferrin, and retinol binding protein.

Particularly preferred plasma proteins with posttranslational modification that are indicative for (early) CDK are listed in Table 2 below. The plasma proteins include all naturally occurring variants and/or splice variants of these proteins.

In an exemplary aspect the method for diagnosing and/or monitoring chronic kidney disease (CKD) in an individual comprises analyzing in vitro the pattern of posttranslational modifications of a plasma protein of the individual, wherein the posttranslational modification is not a folding variant of the serum protein or a mere fragment of the serum protein and wherein where the plasma protein is LDL, the posttranslational modification is not carbamylation and wherein where the plasma protein is haemoglobin or albumin, the posttranslational modification is not carbamylation or oxidation, as further detailed in the claims.

*Table 2: Posttranslational modifications of plasma proteins that are specific for (early) CKD.*

| Serum protein (SEQ ID NO:) | Post-translational modification (Type / Position) |
|---|---|
| **Human Serum Albumin** (HSA; P02768 UniProt) SEQ ID NO:1 | Guanidylation (lysine), positions on K36, K44, K183, K186, K198, K205, K219, K300, K375, K383, K463, K468, K499, K548, K560, K562, K565, K569, K581, K584, K588, K597 and/or K598<br>Glycosylation (2xHex), positions on K28, K375, K460, K499, K548, K565<br>Formylation on K36, K214, K219, K223, K375, K437, K449, K558, K560, K562, K565, K569, K588, K598 |
| **beta-2 microglobulin** (β 2 MG; P61769 UniProt) SEQ ID NO:3 | Oxidation (methionine), position on M119<br>Formylation (N-terminus)<br>Carbamylation (lysine), N-terminus |
| **Cystatin C** (CysC; P01034 UniProt) SEQ ID NO:4 | Formylation (N-terminus)<br>Carbamylation (lysine), (N-terminus) |

(continued)

| Serum protein (SEQ ID NO:) | Post-translational modification (Type / Position) |
|---|---|
| **Transferrin** (P02787 UniProt) SEQ ID NO:5 | Guanidylation (lysine), positions |
| **Retinol binding protein** (P02753 UniProt) SEQ ID NO:6 | Formylation (N-terminus) |

A particularly preferred plasma protein in the context of the present disclosure is serum albumin (Uniprot Accession No. P02768 (ALBU_HUMAN) Reviewed, UniProtKB/Swiss-Prot, last modified September 18, 2013; Version 198). Albumin is a highly important carrier protein of endogenous and exogenous ligands in plasma. Albumin represents the main determinant for the colloid osmotic pressure and the fluid distribution between different body compartments. Albumin is a globular protein containing three different structural domains [Carter et al., Adv Protein Chem. 1994;45:153-203; Deeb et al., Biopolymers. 2011;93:161-170]. This protein is an essential carrier protein for hormones, fatty acids, vitamins and drugs [Fanali et al., Mol Aspects Med. 2012;33:209-290]. Furthermore, albumin has a critical function in the context of CKD [Mahmoodi et al., Lancet. 2012;380:1649-1661; Wada et al., Clin Exp Nephrol. 2012;16:96-101], since albumin is e.g. essential for transportation and detoxification of hydrophobic metabolic compounds like indoxyl sulfate. The binding capacity of albumin for ligands, toxins and metabolites [Lim et al., Nephrology (Carlton). 2007;12:18-24; Gekle et al., J Am Soc Nephrol. 1998;9:960-968; Macconi et al., J Am Soc Nephrol. 2009;20:123-130; Cessac et al., Mech Ageing Dev. 1993;70:139-148] and of indoxyl sulfate (IS) in particular [Watanabe et al., Drug Metab Dispos. 2012;40:1423-1428], is significantly decreased in CKD patients.

While some studies state that the decreased binding capacity, caused by accumulation of endogenous substances leads to competitive inhibition [Klammt et al., Nephrol Dial Transplant. 2012;27:2377-2383; Lorenzo Sellares et al., Nefrologia. 2008;28 Suppl 3:67-78], other studies interpret the altered binding capacity as being due to structural changes of the binding sites caused by conformational changes in the albumin structure [Oettl & Stauber, Br J Pharmacol. 2007;151:580-590]. Although albumin binding of indoxyl sulfate has recently been described [Watanabe et al., Drug Metab Dispos. 2012;40:1423-1428], the impact of post-translational modification on the binding capacity remained unknown.

Without being bound to a theory, the physiological impact of posttranslational modifications on the binding capacity of albumin for e.g. tryptophan and IS, as observed for the first time by the present inventors, gives rise to the expectation that the posttranslational modification of albumin is one factor that leads to the onset of CKD and, at the same time is a factor that aggravates CKD. Based on these observations it is assumed that post-translational modifications of plasma proteins in general affect their biological function and contribute to the onset and the progression of CKD.

A "sample" obtained from an individual to be diagnosed preferably is a blood sample, more preferably a plasma or serum sample. The sample may be obtained freshly prior to the diagnosis or may be stored under conditions that preserve the integrity of the plasma proteins and their posttranslational modification status.

The normal range of glomerular filtration rate (GFR), adjusted for body surface area, is 100-130 ml/min/1.73m$^2$ in men and women. In children, GFR measured by inulin clearance is 110 ml/min/1.73m$^2$ until 2 years of age in both sexes, and then it progressively decreases. After the age of 40 years, the GFR decreases progressively with age, by about 0.4 - 1.2 mL/min per year.

Known risk factors for chronic kidney disease include diabetes, high blood pressure, family history, older age, ethnic group and smoking. Significant proteinuria and urine sediment abnormalities and/or a decline in GFR (decline relative to previous values from the same individual or decline relative to values of a healthy individual) are indicators of kidney disease requiring medical intervention. Chronic kidney disease is classified into different stages (see ICD-10 code N.18), depending on the observed GFR level (see Table 3).

*Table 3: Stages of CDK based on ICD-10 classification.*

| Stage | CKD Stage | |
|---|---|---|
| 0) | Normal kidney function | GFR above 90mL/min/1.73 m$^2$ and no proteinuria |
| 1) | CKD1 | GFR above 90mL/min/1.73 m$^2$ with evidence of kidney damage |
| 2) | CKD2 (Mild) | GFR of 60 to 89 mL/min/1.73 m$^2$ with evidence of kidney damage |
| 3) | CKD3 (Moderate) | GFR of 30 to 59 mL/min/1.73 m$^2$ |
| 4) | CKD4 (Severe) | GFR of 15 to 29 mL/min/1.73 m$^2$ |

(continued)

| Stage | CKD Stage | |
|---|---|---|
| 5) | CKD5 Kidney failure | GFR less than 15 mL/min/1.73 m$^2$ |

The severity of chronic kidney disease (CKD) is usually described by six stages; the most severe three are defined by the MDRD-eGFR (MDRD = Modification of Diet in Renal Disease, eGFR = estimated glomerular filtration rate) value, and first three also depend on whether there is other evidence of kidney disease (e.g., proteinuria).

According to the KDOQI classification, a further stage, CKD5D is attributed to those stage 5 patients requiring dialysis; many patients in CKD5 are not yet on dialysis. In some classifications a "T" is added to describe patients who have had a transplant regardless of stage.

Not all clinicians agree with the above classification, suggesting that it may mislabel patients with mildly reduced kidney function, especially the elderly, as having a disease. A conference was held in 2011 regarding these controversies by Kidney Disease: Improving Global Outcomes (KDIGO) on CKD: Definition, Classification and Prognosis, gathering data on CKD prognosis to refine the definition and staging of CKD (Levey et al., Kidney International (2011) Vol. 80, p. 17-28), see Table 4.

"Pre-disease state CKD" is a state without clinical manifestation of GFR and albuminuria, e.g. the stage G1A1 according to Table 4 below. An individual exhibiting a pre-disease state CKD may develop clinical signs of CKD. Such an individual can also be classified as being "at risk of developing CKD". For example, a patient with diabetes mellitus type I has a risk of developing a CKD of 50%, a patient bearing a gene for developing ADPKD (autosomal dominant polycystic kidney disease) are known to develop CKD, hence their risk for developing a CKD is at 100%. Both of these patients may not yet exhibit any clinical signs of CKD (that is the patient with diabetes mellitus doe not yet have an albuminuria and the patient being diagnosed with ADPKD may not even have a cyst. Further risk factors that are known to lead to CKD are known in the art. Hence, the present invention is particularly suited to monitor patients being diagnosed with any such risk factors.

*Table 4: Composite Ranking for Relative Risks by glomerular filtration rate (GFR) and Albuminuria (KDIGO 2012 Clinical Practice Guideline for the Evaluation and Management of Chronic Kidney Disease. Kidney Int Suppl (2013) 3, 1-150). Colours reflect the ranking of adjusted relative risk. The categories with mean rank numbers 1-8 are green, mean rank numbers 9-14 are yellow, mean rank numbers 15-21 are orange, and mean rank numbers 22-28 are red. Dark red colour for twelve additional cells with diagonal hash marks is extrapolated based on results from the meta-analysis of chronic kidney disease cohorts. The highest level of albuminuria is termed 'nephrotic' to correspond with nephrotic range albuminuria and is expressed here as ≥2000mg/g. Column and row labels are combined to be consistent with the number of estimated GFR (eGFR) and albuminuria stages agreed on at the conference.*

| Composite ranking for relative risks by GFR and albuminuria (KDIGO 2012 Clinical Practice Guideline for the Evaluation and Management of Chronic Kidney Disease. Kidney Int Suppl (2013) 3, 1-150) | | | | Albuminuria stages, Description and range (mg/g) | | | | |
|---|---|---|---|---|---|---|---|---|
| | | | | A1 | | A2 | A3 | |
| | | | | Optimal and high-normal | | High | Very high and nephrotic | |
| | | | | <10 | 10-29 | 30-299 | 300-1999 | ≥2000 |
| GFR stages, description and range (ml/min per 1.73 m$^2$) | G1 | High and optimal | >105 | green | green | yellow | orange | dark red |
| | | | 90-104 | green | green | yellow | orange | dark red |
| | G2 | Mild | 75-89 | green | green | yellow | orange | dark red |
| | | | 60-74 | green | green | yellow | orange | dark red |
| | G3a | Mild-moderate | 45-59 | yellow | yellow | orange | red | dark red |
| | G3b | Moderate-severe | 30-44 | orange | orange | red | red | dark red |
| | G4 | Severe | 15-29 | red | red | red | red | dark red |
| | G5 | Kidney failure | <15 | dark red | dark red | dark red | dark red | dark red |

In another aspect, the present methods can be used to determine whether an individual is at risk of developing a CKD. In such a method an individual will be identified as being at risk of developing CDK, if the individual does not exhibit any clinical signs of CKD as determined by the GFR and the albuminuria stage, but in a serum or plasma sample obtained of the individual the level of posttranslational modification of one post-translationally modified plasma protein specific for CKD is detectably elevated.

An "early stage CKD" is a stage such as G1A2 or G2A1 according to Table 4 above according to the KIDGO classification an early stage CKD can also be associated with urine sediment abnormalities, electrolyte and other abnormalities due to tubular disorders, abnormalities detected by histology, structural abnormalities detected by imaging, or with a history of kidney transplantation.

A "healthy individual", for the sake of the present invention is an individual, which (i) does not exhibit any clinical signs of CKD, (ii) does not exhibit any known risk factors that are known to lead to the development of a CKD (such as ADPKD) and (iii) does not exhibit posttranslational modifications of plasma proteins that are known to be associated with CKD such as the post-translational modifications described hereinabove or -below. For example, a healthy individual or control for the sake of the present invention is an individual exhibiting a GFR of above 90mL/min/1.73 m$^2$, preferably above 100ml/min/1.73 m$^2$ and more preferably above 105mL/min/1.73 m$^2$ and an albuminuria of not more than 10 mg/g, preferably not more than 5 mg/ml, more preferably not more than 1 mg/ml, most preferably the individual does not yet exhibit any measurable albuminuria.

A "control" may be a plasma protein sample obtained from a healthy individual, or a plasma protein produced *in vitro* that is free from posttranslational modifications. Any unmodified plasma protein obtained from a healthy individual can be used as a negative control. Further, any heterogeneously expressed plasma protein can be used as long as the host in which it is expressed does not perform the specific posttranslational modifications outlined above. Further any *in vitro* expressed protein may be used as a negative control, as well as synthetic peptides that correspond to peptides that exhibit a posttranslational modification in post-translationally modified plasma proteins. Correspondingly, a positive control is any protein or peptide that exhibits the specific posttranslational modification(s) outlined above. The source of the protein may be as described for the negative control, the protein or peptide however being chemically modified to exhibit the posttranslational modification(s). Where the plasma protein to be detected is human serum albumin, the positive control is, e.g. *in vitro* guadinylated human serum protein, or human serum protein obtained from a patient being previously diagnosed positively for CKD. The negative control may be recombinantly produced human serum protein from a source that does not perform or exhibit the posttranslational modification of the respective protein or human serum protein obtained from a healthy individual.

As regards "monitoring the progression of CKD". "Progression" is to be understood as developing clinical signs of CKD where those signs were originally absent or were originally present to a lesser degree. In order to monitor the progression of CKD, samples are obtained from an individual to be diagnosed at different points in time. The time between the samples may vary between several days to several weeks or months. The level or extent of post-translationally modified proteins or peptides determined in these samples can be plotted over time and compared to other clinical parameters such as the GFR or the level of albuminurea. Due to the sensitive nature of the underlying methods described hereinabove and below, a progression of CKD can be followed even before other clinical parameters such as the GFR or albuminurea are detectable or are considered to be clinically relevant. This is for example the case where the level of a specific posttranslational modification on a specific plasma protein becomes elevated above its usual base level over time.

An "elevated level" of creatinine and/or albuminurea means a level that is higher than 29 mg/g.

Both glomerular filtration rate (GFR) and the Creatinine clearance rate (Ccr or CrCl) may be accurately calculated by comparative measurements of substances in the blood and urine, or estimated by formulas using just a blood test result (eGFR and eCCr). Methods for determining the GFR and CrCl are known in the art and routinely applied by those of skill in the art (see e.g. Stevens et al. N Engl J Med. 2006 Jun 8;354(23):2473-83). Likewise, the determination of albuminuria can be performed by standard methods (see e.g. de Jong & Curhan, J Am Soc Nephrol 17: 2120-2126, 2006).

"Albuminuria" is a pathological condition wherein albumin is present in the urine; it is a type of proteinuria (for a classification see de Jong & Curhan, J Am Soc Nephrol 17: 2120-2126, 2006).

A "naturally occurring variant" in the context of human serum albumin describes a natural mutant of albumin that has been reported, such as Canterbury (Lys313Asn), Niigata (Asp269Gly), Roma (Glu321Lys), Parklands (Asp365His) and Verona (Glu570Lys), see Kragh-Hansen et al., Eur J Biochem. 1990 Oct 5;193(1):169-74. A full list of naturally occurring variants can be retrieved from Uniprot, Acc. No. P02768 (Last modified November 13, 2013; Version 200.)

MS

Matrix-assisted laser desorption/ionisation (MALDI) mass-spectrometry allows an analysis of post-translational modified proteins, since MALDI-mass-spectrometry produces less multiply charged ions as compared to e.g. electrospray ionization. MALDI-mass spectrometry is an appropriate method for the detection of post-translational modification of digested plasma proteins. For the detection of these modifications by MALDI-mass spectrometry, the cleavage of the protein into peptides is mandatory. The use of trypsin is commonly used for protein digestion in the context of mass-spectrometry. However, if a specific post-translational modification of interest cannot be detected in a tryptic digest, other proteases and cleavage reagents including Lys-C, Glu-C, chymotrypsin and cyanogen bromide can be used, as these produce a complementary set of peptides. Furthermore, post-translational modification on arginine residues (Arg) can inhibit trypsin cleavage. Arg is subject to modification by methylation, dimethylation and citrullination, a stable modification converting the guanidinium group of Arg to an ureido group [Rozman, J Mass Spectrom. 2011; 46:949-955]. Differences in the mass-signal pattern detected within the present invention were at least partly caused by post-translational guanidylation. Quantification of the mass-signal intensities of these peptides demonstrates significant differences in the degree of these post-translational modifications in the control and patient group.

Hence, MALDI-mass-spectrometry is one suitable means for detecting significant differences in the mass-signal pattern of digested plasma proteins (such as e.g. albumin) isolated from healthy controls, CKD patients and patients at risk of developing CKD.

The endogenous post-translational guanidylations of albumin in CKD patients were simulated by an *in vitro* guanidylation of albumin from healthy individuals causing the identical modifications in mass spectra. Hence, in one embodiment, *in vitro* guanidylated plasma proteins can be used as control in those inventive methods that detect the guanidylation status of a plasma protein of an individual to be diagnosed.

Detection of post translational modifications by MALDI-mass spectrometry

Post-translational modifications are detectable by a combination of protein digestion, MALDI mass-spectrometry (MS) MALDI-MS/MS mass-spectrometry and *in-vitro* post-translational modification of proteins.

Firstly, the amino acid sequence of the protein of interest has to be identified by mass-spectrometric fingerprint analysis after tryptic digestion of the protein. Since differences of neighboring mass-signals are caused by cleavages of corresponding peptide bounds, the amino acid sequence of the protein is determinable by calculation of the difference of neighboring mass-signals.

Since mass-signal of the corresponding peptides shifted to an increased mass/charge ratio by post-translational modifications, additional mass/charge-signals are detectable by using MALDI mass-spectrometry in cause of post-translational modified proteins. For example, guanidinylation of the amino acid lysine of a peptide causes a mass/charge-signal shift of 42 Da, formylation of a N-term amino acid of a peptide causes a mass/charge-signal shift of 28 Da, carbamylation of the amino acid lysine of a peptide causes a mass/charge-signal shift of 43 Da or carboxymethylation of amino acids causes a mass/charge-signal shift of 58 Da.

By selective addition of these molecular masses to the mass-signals of unmodified proteins and analyzing the fingerprint mass-spectra of the digested proteins for these potential mass-signals, post-translational modification of proteins are identifiable. For validation of the results of this *in-silico* post-translational modification, the amino acid of the peptide of interested has to be clarified by MALDI-MS/MS experiments. However, the native unmodified protein has to be *in-vitro* post-translationally modified and analyzed by MALDI mass-spectrometry to verify the shift of the mass too.

In addition, the ratio of the mass-signal of the unmodified peptide and the mass-signal of the post-translationally modified peptide reflects the ratio of modified and unmodified peptide amount in analyzed protein mixtures. Therefore, these ratios can be used to calculate the relative amount of the post-translational modification in endogenous proteins.

Antibodies

The present invention also relates to the *in vitro* use of an antibody according to the claims that specifically binds to a post-translationally modified plasma protein. The term "antibody" herein is used in the broadest sense and specifically covers intact monoclonal antibodies, polyclonal antibodies, and antibody fragments so long as they exhibit the desired binding activity. In particular the antibody is a monoclonal antibody. The term "monoclonal antibody" as used herein encompasses any partially or fully human monoclonal antibody independent of the source from which the monoclonal antibody is obtained. A fully human monoclonal antibody is preferred. The production of the monoclonal antibody by a hybridoma is preferred. The hybridoma may be a mammalian hybridoma, such as murine, cattle or human. A preferred hybridoma is of human origin. The monoclonal antibody may also be obtained by genetic engineering and in particular CDR grafting of the CDR segments onto available monoclonal antibodies by replacing the CDR regions of the background antibody with the specific CDR segments as defined in the claims. For example, the monoclonal antibodies to be used in accordance with the present invention may be made by the hybridoma method first described by Kohler et al., Nature, 256:495 (1975), or may be made by recombinant DNA methods (see, e.g., U.S. Pat. No. 4,816,567). The "monoclonal antibodies" may also be isolated from phage antibody libraries using the techniques described in Clackson et al. Nature 352:624-628 (1991) and Marks et al., J. Mol. Biol., 222:581-597 (1991), for example.

The term "specifically binding" in the context of post-translationally modified plasma proteins means that the antibody will specifically bind to the post-translationally modified plasma protein, but not to the plasma protein that lacks the post-translational modification. For example, an antibody specifically binding to guanidylated human serum albumin will specifically bind guanidylated human serum albumin but not human serum albumin that is not guanidylated. Antibodies specific for phosphoserine are known in the art and commercially available from various sources. An antibody may be specific for a particular type of posttranslational modification, e.g. may be specific for guanidylated lysine. That is, an antibody specific for guanidylated lysine will be capable to detect guanidylated lysine in either human serum albumin or β2-MG or transferrin, or any other plasma protein that is guadinylated at a lysine position. Hence, the detection of whether a specific posttranslational modification of a particular plasma protein is present can be performed as follows: (i) identifying the nature of the plasma protein of interest, e.g. with an antibody specific for the protein and (ii) immunologically determining whether the specific posttranslational modification is present in that protein (e.g. by consecutively probing a Western blot or by stripping and reprobing a Western blot). How the nature of the plasma protein is determined is not critical as long as the identification allows for a subsequent analysis of the posttranslational modification status of the protein.

Hence, antibodies that specifically bind to an epitope that as its major immunological determinant comprises the respective post-translationally modified amino acid residue, largely irrespective of the remainder of the immunogenic peptide will be specific for the type of posttranslational modification and not specific for the respective plasma protein. the present invention relates to the *in vitro* use of antibodies according to the claims that specifically detect a post-translationally modified amino acid only in conjunction with an epitope that is specific for the respective plasma protein.

In other words, such antibodies will be specific for only detecting a posttranslational modification on a specific plasma protein.

Preferably an antibody specifically detects the posttranslational modification specified in Table 2 above, e.g. the antibody will detect a modification selected from the guanidylation of albumin (more preferably human serum albumin), the guanidylation of β2MG (preferably on at least one lysine residue), the oxidation of β2MG (preferably, on at least one methionine residue), the formylation of β2MG (preferably on the N-terminus of β2MG), the carbamidomethylation of β2MG (preferably on at least one cysteine residue), the carbamylation of β2MG (preferably on at least one lysine residue), the carboxymethylation of β2MG (preferably on at least one cysteine residue), the formylation of cystatin C (preferably on the N-terminus), the carboxymethylation of cystatin C (preferably on at least one cysteine residue), the carbamylation of cystatin C (preferably on at least one lysine residue), the guanidylation of transferrin (preferably on at least one lysine residue), and the formylation of retinol binding protein (preferably on the N-terminus).

A skilled person will appreciate that the $K_D$ of the antibody with respect to the post-translationally modified plasma protein is $10^{-6}$ or lower, preferably $10^{-7}$ or lower. Said antibody does not specifically bind to the plasma protein which is not post-translationally modified, i.e. the antibody binds to a plasma protein of an individual afflicted with CKD or exhibiting a condition that may develop into CKD, while the antibody does not bind to the same plasma protein obtained from a healthy individual (not suffering from CKD, and/or not being susceptible of developing CKD). Suitably, the $K_D$ of the antibody with respect to the plasma protein that is not post-translationally modified is $5 \times 10^{-5}$ or higher.

The term "specifically binding a target protein" means that the antibody binds to target protein with a higher affinity as compared to the binding to any other protein. A skilled person would understand that an unspecific binding to non-target proteins may occur as an artefact.

The term "CDR region" means the complementarity determining region of an antibody, i.e. the region determining the specificity of an antibody for a particular antigen. Three CDR regions (CDR1 to CDR3) on both the light and heavy chain are responsible for antigen binding. The term "fragment" means any fragment of the antibody capable of specifically binding to the post-translationally modified plasma protein (e.g. to the guanilydated from of serum albumin). It is further preferred that the fragment used comprises the binding region of the antibody. Examples of antibody fragments include Fab, Fab', F(ab')2, and Fv fragments; diabodies; linear antibodies and single-chain antibody molecules. It is preferred that the fragment used is a Fab, F(ab')$_2$, single chain or domain antibody. Most preferred is the use of a Fab or F(ab')$_2$ fragment or a mixture thereof.

Accordingly, a monoclonal antibody as defined herein is used in vitro in the claimed methods, wherein the antibody is a Fab, F(ab')$_2$, single chain or domain antibody fragment.

The antibodies discussed above may be used to detect posttranslational modifications on serum proteins in various analytical methods such as Western Blot or ELISA.

By way of example, an antibody specifically binding a post-translationally modified protein can be generated by using the *in vitro* modified plasma protein as an antigen. Alternatively, post-translationally modified proteins obtained from individually suffering from CKD may be used as an antigen after appropriate purification of these proteins. Likewise, only one or more specific peptides of a selected plasma protein that exhibit the posttranslational modification can be used as an antigen for immunization. In the latter case it may be necessary to crosslink or polymerize the peptides in order to make them more immunogenic. Antibodies that are specific for a post-translationally modified protein or peptides may be obtained by screening hybridoma cell lines for binding to the post-translationally modified plasma protein or peptide using competitive ELISA with non-modified plasma protein or peptide as competing agent. Specific subfractions from polyclonal antisera may be obtained by affinity purification using post-translationally modified plasma proteins or peptides as solid phase (e.g. linked to NHS-Sepharose).

ELISA

Enzyme-linked immunosorbent assays (ELISAs) for various antigens include those based on colorimetry, chemiluminescence, and fluorometry. ELISAs have been successfully applied in the determination of low amounts of drugs and other antigenic components in plasma and urine samples, involve no extraction steps, and are simple to carry out. Suitable ELISA techniques are direct ELISA, indirect ELISA and sandwich ELISA.

Antibodies can be used as capture antibodies that are immobilized and capture post-translationally modified proteins or peptides. The so immobilized post-translationally modified proteins or peptides can then be detected by antibodies specific for the respective protein or peptide.

Alternatively, the antibody may be modified (e.g. conjugated to a detectable label such as a fluorochrome or to an enzyme), preferably the antibody is conjugated to horseradish peroxidase (HRP), alkaline phosphatase (AP) or biotin. In this setting, the post-translationally modified protein or peptide is immobilized to the solid phase of the ELISA by conventional means, and the antibody is used as a secondary antibody.

The antibody added to the immobilized capture reagents will be either directly labelled, or detected indirectly by addition, after washing off of excess first antibody, of a molar excess of a second, labelled antibody directed against IgG of the animal species of the first antibody. In the latter, indirect assay, labelled antisera against the first antibody are added to the sample so as to produce the labelled antibody *in situ.*

The basic ELISA procedure is known to skilled person and described more specifically in e.g. "The ELISA Guidebook", Methods in Molecular Biology Vol. 149, John R. Crowther, Humana Press, 2000.

Enzyme assays, binding assays

In one aspect the present invention's rationale is based on the characterisation in detail of the physiological and pathophysiological effect of the identified post-translational modification of the plasma proteins. In one embodiment of this aspect the effects of the post-translational modification of albumin on the binding of hydrophobic metabolites in human plasma can be determined. In these binding studies, albumin and ligand concentration as possible reason for altered binding can be excluded. Comparing the protein bound fraction of albumin isolated from healthy control subjects and CKD patients, competitive ligand binding of hydrophobic plasma compounds can also be dismissed as the cause for the altered binding capacity of albumin in CKD patients.

Thus, the present inventors have shown that post-translational modifications of the protein are involved which result in either structural alterations of the binding pocket or conformational changes distant from the binding site but influencing the binding of hydrophobic plasma compounds to albumin. Using the MALDI-mass-spectrometric method, significant differences in the mass-signal pattern of tryptic albumins peptides isolated from healthy controls and CKD patients were detected.

Further information can be obtained from a detailed analysis of the binding curves of CKD patients and of albumin from healthy controls before and after *in vitro* guanidylation. While binding of IS to albumin of healthy individuals follows the typical binding curve with specific binding at low ligand concentration slowly progressing to non-specific binding for ligand concentrations > -20 $\mu$M, albumin from CKD patients and albumin after *in vitro* guanidylation are very similar showing little specific and almost exclusively non-specific binding. Thus, the dissociation constant $K_D$ of albumin was not significantly affected by post-translational modifications (6.01 $\pm$ 1.83 $\mu$M vs. 2.71 $\pm$ 0.79 $\mu$M) and was, also after modification, in the same range, i.e. between 1-10 $\mu$M, known from literature. In contrast, the number of specific binding sites ($B_{max}$) of post-translational modified albumin was significantly decreased (27.45 $\pm$ 3.97 $\mu$M vs. 8.58 $\pm$ 3.18 $\mu$M) as compared to unmodified albumin. The stronger decrease of binding capacity for L-tryptophan upon *in vitro* guanidylation compared to indoxyl sulfate reflects the more than two orders of magnitude lower affinity of L-tryptophan to post-translational modified albumin as compared to indoxyl sulfate [Irikura et al., Chem Pharm Bull (Tokyo). 1991; 39:724-728; Kragh-Hansen, 1 Biochem J. 1991;273 (Pt 3):641-644; Sarnatskaya et al. Biochem Pharmacol. 2002;63:1287-1296].

The modifications of the human serum albumin at lysines 36, 183, 198, 569 and 581 led to decrease of indoxyl sulfate binding. None of these residues are located in binding site I and II. Thus we conclude, that guanidylation at these positions lead to a conformational change of albumin resulting in lower binding affinity by an allosteric mechanism.

The difference in the amount of protein-bound indoxyl sulfate by albumin isolated from healthy control subjects compared to CKD patients demonstrates the *in-vivo* relevance of post-translational guanidylation. The decrease in the maximum number of specific binding sites has a direct and negative effect on the carrier capacity of albumin. Since the carrier capacity of albumin is essential for both, detoxification and hemostasis of the organism, preventive treatments in order to avoid post-translational modification of plasma proteins are a highly relevant approach against progression of disease.

The present disclosure reveals quantitatively the impact of post-translational modification on the physiological properties of human serum albumin (HSA). The present disclosure enables a precise correlation between the binding capacity of albumin and its post-translational modification status. This makes it possible to monitor the progression of CKD by testing the binding characteristics of HSA with indoxyl sulfate and tryptophan.

**MATERIAL AND METHODS**

Human albumin, heparin, ammonium formiate, O-methyl isourea bisulfate, indoxyl sulfate potassium salt, L-tryptophan, PBS and bis-tris-propane were obtained from Sigma Aldrich (Germany). Acetonitrile and sodium chloride were obtained from Merck Chemicals (Germany).

ISOLATION OF ALBUMIN FROM HEALTHY CONTROL SUBJECTS AND CKD PATIENTS

Study approval was obtained from the local ethics committee of the medical faculty of the University of Würzburg, Germany (no. 64/12). For isolation of albumin from plasma of healthy control subjects, 400 ml heparinzed (5 U ml-1) blood was obtained by venepuncture and plasma was isolated from blood by centrifugation (2,000 $\times$ g, 50 min, RT). Protein aggregates were removed from plasma by dead end filtration (6 ml min-1, RT) using a microporous membrane (MicroPES TF 10; 175 cm$^2$ inner surface area, Membrana GmbH Germany). Plasma proteins were isolated by dead-end filtration (20 ml min$^{-1}$, RT) using a plasma fractionation membrane with a nominal cut-off of 250 kDa (FractioPES 50; 1.77 m$^2$ inner surface area, Membrana GmbH, Germany). Albumin of CKD patients (stage 5D) was isolated from haemofiltrate concentrated using Cuprophan membranes (1.2 m$^2$, steam-sterilized dialyzer, Haidylena, Egypt) and in a second step by an ultrafiltration unit using a ultrafiltration membrane (regenerated cellulose, MWCO 1000 Da, Millipore) (Amicon, Germany). After a dialysis step against 6.25 mmol l$^{-1}$ bis-tris-propane-buffer (pH 7.5), the albumin-containing concentrates were applied to an anion-exchange column (Sepharose Fast Flow Q; GE Healthcare, Germany) equilibrated in the same buffer (flow rate: 1.6 ml min$^{-1}$). 6.25 mmol ml$^{-1}$ bis-tris-propane-buffer (pH 7.5) was used as eluent A and an aqueous solution of 0.35 mmol l$^{-1}$ NaCl (pH 9.5) as eluent B using a linear gradient: 0-100%B in 1080 min, UV detection at $\lambda_{280nm}$. The albumin-containing fraction was lyophilised, resuspended in water and fractionated by size-exclusion chromatography (Sephacryl S200 column; GE Healthcare, Germany) using a phosphate-buffered solution (138.0 mmol l$^{-1}$ NaCl, 2.7 mmol l$^{-1}$ KCl at pH 7.4 and 25°C, flow rate of 0.2 ml min$^{-1}$, UV detection at $\lambda_{280nm}$).

The homogeneity of the isolated albumin containing fractions was analysed by SDS-PAGE chromatography. Briefly, SDS-PAGE was performed at room temperature according to Laemmli's procedure under reducing conditions [Laemmli, Nature. 1970; 227:680-685]. All reagents for electrophoretic analyses were obtained from Bio-Rad (Germany) performed in vertical slab gels containing a 4-20 % gradient of total acrylamide (Criterion Tris-HCl-Precast gel, Bio-Rad, Germany). An external voltage power supply was used (EPS 301; GE Healthcare, Germany) at 120V for 90 min. Gels were stained for 60 min with Coomassie Brilliant Blue R-250 (Bio-Rad (Germany)) and destained with Coomassie Brilliant Blue R-250 destaining solution (Bio-Rad, Germany) over night. The Gel-X-Pro-Analyzer software was used data for analysis (version 3.1, Media Cybernetics, USA). To exclude artificial modification of the albumin during the entire separation procedure, unmodified albumin (Sigma-Aldrich, Germany) was applied to the isolation process three times in a row and analysed as described below.

IDENTIFICATION OF POST-TRANSLATIONAL MODIFICATION OF ALBUMIN BY MALDI-MASS-SPECTROMETRY

Isolated albumin was digested by trypsin and then analysed by matrix assisted laser desorption/ionisation time of flight mass-spectrometry (MALDI TOF/TOF-mass-spectrometry). First, the albumin fractions were incubated with aqueous ammonium bicarbonate (50 mmol l$^{-1}$) and 0.2% w/c trypsin of 24 h at 37°C. The tryptic albumin peptides were concentrated and desalted utilizing the ZipTip$_{C18}$ (Millipore, USA) technology using water with 0.1% trifluoroacetic acid (TFA) and 80% acetonitrile (ACN) in water. The eluate was spread onto the MALDI target plate (MTP-AnchorChip 400/384; Bruker-Daltonic, Germany) using $\alpha$-cyano-4-hydoxycinamic acid (2.5 mg ml$^{-1}$) as matrix by using a robot (Freedomevo, Tecan, Switzerland). The subsequent mass-spectrometric analyses were carried out using a reflectron-type time-of-flight-mass spectrometer MALDI-TOF/TOF mass-spectrometer (Ultraflex III, Bruker-Daltronic, Germany). MS/MS were accumulated by using the Lift-option of the mass-spectrometer. The mass-spectra were calibrated and annotated by using BioTools 3.2, Bruker, Germany) in combination with the MASCOT 2.2 database (Matrix Science; London (UK)) comparing experimental mass-spectrometric data with calculated peptide masses for each entry in the sequence database.

*IN VITRO* GUANIDYLATION OF ALBUMIN

*In-vitro* guanidylation of albumin of healthy control subjects was performed as described [Kimmel, Meth. Enzymology. 1967:584]. Briefly, 200 mg ml$^{-1}$ albumin solution was mixed with 1 mol l$^{-1}$ o-methylisourea-bisulfate solution (pH 11.0) to reach a final concentration of 1.5 mmol l$^{-1}$ albumin in 0.5 mol l$^{-1}$ o-methyl isourea solution (0°C). At different time points (0, 3, 6, 24, 48 h) the reaction mixture was diluted and dialyzed against PBS. Binding studies of indoxyl sulfate and L-tryptophan were done as described below.

DETERMINATION OF PROTEIN BOUND FRACTION OF ALBUMIN

For the determination of the protein bound fraction of hydrophobic plasma compounds to albumin, indoxyl sulfate (7.5 $\mu$mol l$^{-1}$) and L-tryptophan (7.5 $\mu$mol l$^{-1}$), respectively, were incubated with albumin (75 $\mu$mol l$^{-1}$) for 30 min at room temperature. After incubation, for the determination of unbound (free) ligand concentration, the mixture was centrifuged by using a centrifugal filter with a cut-off of 30 kDa (VWR, Germany) for 15 min at 10,000 $\times$ g at room temperature. In a parallel approach, the incubation mixture was heated at 95 °C for 30 min for determination of total (unbound and bound) ligand concentration. The mixture was centrifuged using the identical conditions as described above.

Amount of unbound and total indoxyl sulfate and L-tryptophan in the filtrate was quantified by reversed-phase chromatography using a C$_{18}$ column (Prontosil Hypersorb ODS, Bischoff Chromatography, Germany). 50 mmol l$^{-1}$ ammonium formate (pH 3.4) was used as eluent A; 100 % acetonitrile as eluent B. The substances, retained by the reversed-phase gel, were eluted using a linear gradient: 0-3.5 min, 15 % eluent B, 3.5-10 min, 15-25 % eluent B, 10-12 min, 25-100 % eluent B and 12-15 min, 100 % eluent B. The flow rate was 1 ml min$^{-1}$ and the temperature 30 °C. The elution was monitored with fluorescence at $\lambda_{Exc}$ 280 nm and $\lambda_{Em}$ 340 nm. The Chromeleon software (version 6.8; Dionex; Idstein (Germany)) was used for data acquisition and quantitation. The amount of protein-bound fraction of indoxyl sulfate or L-tryptophan ($c_{bound}$) was calculated from the total amount ($C_{total}$) and the unbound amount ($C_{free}$) using the following equation:

$$C_{bound} = \frac{C_{total} - C_{free}}{C_{total}} * 100$$

with $c_{bound}$ in [%] and $C_{total}$ and $C_{free}$ in [$\mu$mol L$^{-1}$] each.

DETERMINATION OF DISSOCIATION CONSTANT AND NUMBER OF BINDING SITES FOR INDOXYL SULFATE

Albumin (37.5 $\mu$mol l$^{-1}$) in PBS was incubated for 30 min at RT with indoxyl sulfate (0.9; 2.8; 3.8; 18.8; 37.5; 75.0; 112.5; 150.0 $\mu$mol l$^{-1}$, each) for determination of dissociation constant ($K_d$) and maximal number of specific binding sites ($B_{max}$). Unbound and total indoxyl sulfate was quantified by HPLC. $K_d$ and the $B_{max}$ values were determined using the GraphPad Prism (V5.0; GraphPad Software, USA) software utilizing the one site total binding equation. For calculation of the specific binding, non-specific binding was subtracted from the total binding.

*IN-VITRO* DIALYSIS OF ALBUMIN ISOLATED FROM CKD PATIENTS AND HEALTHY CONTROL SUBJECTS

Albumin (75 $\mu$mol l$^{-1}$, 1 ml) from CKD patients and healthy control subjects by anion-exchange and size exclusion chromatography was dissolved in PBS, added to a Slide-A-Lyzer Dialysis Cassettes (cut-off 3.5 kDa, Thermo Scientific, USA) and then dialyzed against 1 l of 1 mol l$^{-1}$ NaCl at 2-8°C. After 24, 48 and 96 h, samples were desalted by dialysis against PBS for 24 h. Binding capacity was determined as described above using 75 $\mu$mol l$^{-1}$ albumin and 7.5 $\mu$mol l$^{-1}$ indoxyl sulfate.

STATISTICAL ANALYSIS

Data are expressed as mean $\pm$ SD. Impaired Student's *t*-test was used for statistical analysis (GraphPad Prism, V5.0; GraphPad Software, USA) A p-value of <0.05 was considered to be significant.

## Examples

The following examples are illustrating specific embodiments of the present invention and are not to be construed so as to limit the scope of the invention as defined in the appended claims.

EXAMPLE 1

**Figure 1** shows representative mass-fingerprint-spectra of albumin isolated from the plasma of healthy control subjects (**Figure 1A**) and albumin from CKD patients (**Figure 1B**) both after digestion with trypsin. The mass-spectra of albumin isolated from the CKD patients showed additional mass-signals compared to albumin from the healthy individual (differences labelled by grey arrows in **Figure 1B**). Characteristic mass-signals were further analysed by MS/MS mass-spectrometry for determination of the underlying molecular differences.
**Figure 1C** provides a typical MS/MS mass-spectrum of the tryptic fragment of 817.19 Da of albumin isolated from healthy control subjects. The underlying amino acid sequence was identified by data-alignment of the MS/MS-data using the Mascot database 2.2 (Matrix Science, London, UK). The corresponding representative MALDI MS/MS mass-spectrum of the tryptic fragment of 858.54 Da of albumin from CKD patients is shown in **Figure 1D.**
The amino acid sequence of albumin contains 609 amino acids [UniProt P02768, SEQ ID NO:1]. Analyses of the MS/MS data using the Mascot database as well as by analysing the MS/MS data using a *de-novo* approach demonstrate that differences of MS/MS fragments were caused by post-translational modification of lysines; this is indicated by the asterisk in **Figure 1D. Figure 1E** shows the corresponding quantification of lysine guanidylation in CKD patients (black bars) compared to unmodified lysines in healthy control subjects (grey bars, mean of n=4 samples).

EXAMPLE 2

The amino acid sequence of albumin is shown on **Figure 2A**. Red boxes indicate guanidylated lysines of albumin detected in both, modified albumin from CKD patients and in albumin after *in-vitro* guanidylation. The modification of the protein at positions 36, 183, 198, 569 and 581 belongs to fragments with mass signals at 860, 916, 715, 488, 999 Da. The mass-spectrum of unmodified albumin after digestion with trypsin is shown in **Figure 2B**. *In-vitro* guanidylation of this albumin leads to the same extent of guanidylation as compared to albumin isolated from CKD patients (**Figure 2C**). Both modified albumins show impaired binding of indoxyl sulfate (data not shown).

To clarify the pathophysiological impact of albumin guanidylation, we investigated the binding capacity of *in-vitro* guanidylated albumin for indoxyl sulfate as a representative hydrophobic metabolic waste product. **Figure 2D** shows the decreased binding of albumin for indoxyl sulfate with increased *in-vitro* guanidylation over time (* $p < 0.01$; ** $p < 0.005$). *In-vitro* guanidylation of the albumin leads to a decrease in the total binding of albumin for indoxyl sulfate (■ of **Figure 2E** vs. ■ of **Figure 2F**). The decreased total binding results from both, decreased specific (▼) and unspecific (♦) binding of indoxyl sulfate to albumin. The number of specific binding sites ($B_{max}$) of albumin for indoxyl sulfate decreased upon guanidylation from $17.52 \pm 0.75$ $\mu$mol l$^{-1}$ to $6.28 \pm 1.42$ $\mu$mol l$^{-1}$.

EXAMPLE 3

Next, we demonstrated a significantly decreased protein bound fraction of indoxyl sulfate to albumin from CKD patients (**Figure 3A**) being $0.88 \pm 0.03$ in healthy control subjects vs. $0.74 \pm 0.1$ in CKD patients (mean of n=6 controls and n=5 patients). To remove endogeneous hydrophobic compounds from albumin of CKD patients, we purified albumin first by size-exclusion chromatography and afterwards by intense dialysis against 1 mol l$^{-1}$ NaCl. Aliquots of albumin before and after size-exclusion chromatography as well as after dialysis were further analysed by reversed-phase chromatography (**Figure 3B**). Size-exclusion chromatography (SEC) caused a significant release of hydrophobic plasma compounds from albumin (chromatogram I vs. II of **Figure 3B**). The chromatograms after SEC and subsequent dialysis showed no fluorescence of hydrophobic plasma compounds (chromatogram I vs. III of **Figure 3B)**. Albumin isolated from the plasma of healthy control subjects was purified in the same manner (**data not shown**). Finally, we quantitatively compared the binding capacity of albumin from healthy controls and of albumin from CKD patients for indoxyl sulfate. Although intense dialysis partially increased the protein bound fraction of both albumin preparations, the protein bound fraction of albumin from CKD patients (black bars in **Figure 3C**) was still significantly decreased when compared to albumin from healthy controls (grey bars in **Figure 3C**)).

EXAMPLE 4

To verify the results of the *in-vitro* guanidylation on albumin binding properties as shown in **Figure 2E and 2F** we analyzed albumin from healthy control subjects (**Figure 4A**) and CKD patients (**Figure 4B**) for indoxyl sulfate binding in more detail. Post-translational modification of albumin in CKD patients had a strong impact on total (■), specific (▼) and unspecific (♦) binding of albumin compared to albumin from healthy controls. The dissociation constants ($K_d$) of albumin isolated from healthy controls and CKD patients were not significantly different ($6.10 \pm 1.83$ vs $2.71 \pm 0.79$ $\mu$mol l$^{-1}$; **Figure 4C**). However, the number of specific binding sites ($B_{max}$) are significantly decreased in CKD patients ($27.45 \pm 3.97$ vs. $8.58 \pm 3.18$ pmol l$^{-1}$) (**Figure 4C**).

Finally, we analysed the impact of post-translational guanidylation on the albumin binding of L-tryptophan, a physiologically highly relevant nutrient. Also *in-vitro* guanidylation of albumin causes a strong decrease of the binding of modified albumin for L-tryptophan (**Figure 4D**). The effect caused by guanidylation was highly correlated over time of guanidylation with the effect on the binding for indoxyl sulfate (**Figure 2D**). However, the effect was stronger for L-tryptophan (a decrease of 81 %) as compared to indoxyl sulfate (decrease of 32 %). Finally, to check whether modifications are generated by chemicals or procedures of the analytical method, unmodified commercial albumin was applied to the isolation process described in the Material and Methods section three times, consecutively. We did not detect any modification by this procedure.

## Claims

1. A method for diagnosing and/or monitoring early stage chronic kidney disease (CKD) in an individual, the method comprising:

   (a) analyzing *in vitro* the pattern of posttranslational modifications of a plasma protein of an individual to be diagnosed, wherein the plasma protein is selected from the group consisting of albumin, beta-2 microglobulin (β2MG), cystatin C, transferrin, and retinol binding protein; wherein the posttranslational modification is selected from the group consisting of guanidylation of one or more lysine residue(s) of human serum albumin selected from positions K36, K44, K183, K186, K198, K205, K219, K300, K375, K383, K463, K468, K499, K548, K560, K562, K565, K569, K581, K584, K588, K597 and/or K598 as depicted in SEQ ID No. 1; glycosylation 2xHex, i.e., addition of a glucose molecule to one or more lysine residue(s) of human serum albumin selected from positions K28, K375, K460, K499, K548, K565 as depicted in SEQ ID No. 1, and subsequent addition of a further glucose molecule to the first glucose molecule; formylation of one or more lysine residue(s) of human serum albumin selected from K36, K214, K219, K223, K375, K437, K449, K558, K560, K562, K565, K569, K588, K598 as depicted in SEQ ID No. 1; oxidation of methionine 119 of beta-2 microglobulin as depicted in SEQ ID No. 3; formylation of the N-terminus of beta-2 microglobulin, carbamylation of a lysine residue or the N-terminus of beta-2 microglobulin; formylation of the N-terminus of Cystatin C, carbamylation of the N-terminus or lysine of Cystatin C; guanidylation of a lysine of transferrin; and formylation of the N-terminus of retinol binding protein;

(b) comparing the posttranslational modification pattern of the plasma protein of the individual to be diagnosed with the posttranslational modification pattern of the plasma protein of an individual not suffering from CKD;

(c) detecting a difference in the posttranslational modification pattern of the plasma protein of the individual to be diagnosed as compared to the posttranslational modification pattern of the plasma protein from the individual not suffering from CKD; and

(d) diagnosing CKD, if the plasma protein of the individual to be diagnosed exhibits at least one posttranslational modification according to a) above that is absent in the plasma protein of the individual not suffering from CKD.

2. The method of claim 1, wherein the individual to be diagnosed does not exhibit an elevated level of creatinine and/or albuminurea, preferably the individual to be diagnosed exhibits a glomerular filtration rate (GFR) of >90mL/min.

3. The method of any one of claims 1 or 2, wherein the plasma protein has an amino acid sequence of any one of SEQ ID NO 1, 2, 3, 4, 5 or 6.

4. The method of any one of claims 1 to 3, wherein the plasma protein is human serum albumin, preferably human serum albumin of SEQ ID NO:1.

5. The method of any one of claims 1 to 4, wherein the detecting of the difference in the posttranslational modification pattern of the plasma protein is performed by mass spectrum analysis of the isolated plasma protein, or ELISA, Western Blot, 2D page following colorimetric detection of the post-translational modification.

6. In vitro use of an antibody in a method according to any of claims 1 to 5, wherein the antibody specifically binds to a post-translationally modified plasma protein selected from the group consisting of albumin, beta-2 microglobulin (β2MG), cystatin C, transferrin, and retinol binding protein, wherein the plasma protein preferably has an amino acid sequence of any one of SEQ ID NO 1, 2, 3, 4, 5 or 6; wherein the posttranslational modification is selected from the group consisting of guanidylation of one or more lysine residue(s) of human serum albumin selected from positions K36, K44, K183, K186, K198, K205, K219, K300, K375, K383, K463, K468, K499, K548, K560, K562, K565, K569, K581, K584, K588, K597 and/or K598 as depicted in SEQ ID No. 1; glycosylation 2xHex of one or more lysine residue(s) of human serum albumin selected from positions K28, K375, K460, K499, K548, K565 as depicted in SEQ ID No. 1; formylation of one or more lysine residue(s) of human serum albumin selected from K36, K214, K219, K223, K375, K437, K449, K558, K560, K562, K565, K569, K588, K598 as depicted in SEQ ID No. 1; oxidation of methionine 119 of beta-2 microglobulin as depicted in SEQ ID No. 3; formylation of the N-terminus of beta-2 microglobulin, carbamylation of a lysine residue or the N-terminus of beta-2 microglobulin; formylation of the N-terminus of Cystatin C, carbamylation of the N-terminus or lysine of Cystatin C; guanidylation of a lysine of transferrin; and formylation of the N-terminus of retinol binding protein;

wherein the antibody does not specifically bind to the plasma protein which is not post-translationally modified in an individual not suffering from CKD.

7. The in vitro use of an antibody of claim 6, wherein the plasma protein is human serum albumin.

8. The in vitro use of an antibody of claim 6 or 7, wherein the post-translational modification is guanidylation.

9. The method of any one of claims 1 to 5, wherein the difference in the posttranslational modification pattern of the plasma protein of the individual to be diagnosed is detected using the antibody used according to any one of claims 6 to 8.

**Patentansprüche**

1. Ein Verfahren zur Diagnose und/oder zum Monitoring einer chronischen Nierenerkrankung (CKD) im Frühstadium in einem Individuum, das Verfahren umfassend:

(a) *in vitro* Analyse des Musters von posttranslationalen Modifikationen eines Plasmaproteins eines zu diagnostizierenden Individuums, wobei das Plasmaprotein ausgewählt ist aus der Gruppe bestehend aus Albumin, beta-2 Microglobulin (β2MG), Cystatin C, Transferrin, und Retinolbindendes Protein; wobei die posttranslationale Modifikation ausgewählt ist aus der Gruppe bestehend aus Guanidylierung eines oder mehrerer Lysinrest(e/s) von wie in SEQ ID NO. 1 abgebildetem humanem Serumalbumin an Positionen ausgewählt aus K36, K44, K183, K186, K198, K205, K219, K300, K375, K383, K463, K468, K499, K548, K560, K562, K565, K569, K581, K584, K588, K597 und/oder K598; Glycosylierung 2xHex, d.h. Addition eines Glucosemoleküls an einen oder mehrere Lysinrest(e) von wie in SEQ ID NO. 1 abgebildetem humanem Serumalbumin an Positionen ausgewählt aus K28, K375, K460, K499, K548, K565, und nachfolgende Addition eines weiteren Glucosemoleküls an das erste Glucosemolekül; Formylierung eines oder mehrerer Lysinrest(e/s) von wie in SEQ ID NO. 1 abgebildetem humanem Serumalbumin an Positionen ausgewählt aus K36, K214, K219, K223, K375, K437, K449, K558, K560, K562, K565, K569, K588, K598; Oxidation von Methionin 119 von wie in SEQ ID NO:3 abgebildetem beta-2 Microglobulin; Formylierung des N-Terminus von beta-2 Microglobulin; Carbamylierung eines Lysinrests oder des N-Terminus von beta-2 Microglobulin; Formylierung des N-Terminus von Cystatin C; Carbamylierung des N-Terminus oder Lysin von Cystatin C; Guanidylierung eines Lysins von Transferrin; und Formylierung des N-Terminus von Retinolbindendem Protein;

(b) Vergleichen des posttranslationalen Modifikationsmusters des Plasmaproteins des zu diagnostizierenden Individuums mit dem posttranslationalen Modifikationsmuster des Plasmaproteins eines Individuums, das nicht an CKD leidet;

(c) Erfassen eines Unterschieds im posttranslationalen Modifikationsmuster des Plasmaproteins des zu diagnostizierenden Individuums im Vergleich zum posttranslationalen Modifikationsmuster des Plasmaproteins des Individuums, das nicht an CKD leidet; und

(d) Diagnostizieren von CKD, wenn das Plasmaprotein des zu diagnostizierenden Individuums mindestens eine posttranslationale Modifikation gemäß (a) aufweist, die in dem Plasmaprotein des Individuums, das nicht an CKD leidet, fehlt.

2. Das Verfahren nach Anspruch 1, wobei das zu diagnostizierende Individuum keinen erhöhten Kreatinin- und / oder Albuminharnstoffspiegel aufweist, vorzugsweise weist das zu diagnostizierende Individuum eine glomeruläre Filtrationsrate (GFR) von > 90 ml / min auf.

3. Das Verfahren gemäß einem beliebigen der Ansprüche 1 oder 2, wobei das Plasmaprotein eine Aminosäuresequenz von einer beliebigen der SEQ ID NOs 1, 2, 3, 4, 5 oder 6 aufweist.

4. Das Verfahren nach einem beliebigen der Ansprüche 1 bis 3, wobei das Plasmaprotein humanes Serumalbumin, vorzugsweise humanes Serumalbumin der SEQ ID NO: 1 ist.

5. Das Verfahren nach einem beliebigen der Ansprüche 1 bis 4, wobei der Nachweis des Unterschieds im posttranslationalen Modifikationsmuster des Plasmaproteins durch Massenspektrumanalyse des isolierten Plasmaproteins oder ELISA, Western Blot, oder 2D-PAGE gefolgt von kolorimetrischem Nachweis der posttranslationalen Modifikation durchgeführt wird.

6. *In vitro* Verwendung eines Antikörpers in einem Verfahren gemäß einem beliebigen der Ansprüche 1 bis 5, wobei der Antikörper spezifisch an ein posttranslational modifiziertes Plasmaprotein bindet, ausgewählt aus der Gruppe bestehend aus Albumin, beta-2 Microglobulin (β2MG), Cystatin C, Transferrin, und Retinolbindendes Protein; wobei das Plasmaprotein vorzugsweise eine Aminosäuresequenz von einer beliebigen der SEQ ID NOs 1, 2, 3, 4, 5 oder 6 aufweist; wobei die posttranslationale Modifikation ausgewählt ist aus der Gruppe bestehend aus Guanidylierung eines oder mehrerer Lysinrest(e/s) von wie in SEQ ID NO. 1 abgebildetem humanem Serumalbumin an Positionen ausgewählt aus K36, K44, K183, K186, K198, K205, K219, K300, K375, K383, K463, K468, K499, K548, K560, K562, K565, K569, K581, K584, K588, K597 und/oder K598; Glycosylierung 2xHex, d.h. Addition eines Glucosemoleküls an einen oder mehrere Lysinrest(e) von wie in SEQ ID NO. 1 abgebildetem humanem Serumalbumin an Positionen ausgewählt aus K28, K375, K460, K499, K548, K565, und nachfolgende Addition eines weiteren Glucosemoleküls an das erste Glucosemolekül; Formylierung eines oder mehrerer Lysinrest(e/s) von wie in SEQ ID NO. 1 abgebildetem humanem Serumalbumin an Positionen ausgewählt aus K36, K214, K219, K223, K375, K437, K449, K558, K560, K562, K565, K569, K588, K598; Oxidation von Methionin 119 von wie in SEQ ID NO:3 abgebildetem beta-2 Microglobulin; Formylierung des N-Terminus von beta-2 Microglobulin; Carbamylierung eines Lysinrests oder des N-Terminus von beta-2 Microglobulin; Formylierung des N-Terminus von Cystatin C; Carbamylierung des N-Terminus oder Lysin von Cystatin C; Guanidylierung eines Lysins von Transferrin; und Formylierung des N-Terminus von Retinolbindendem Protein;
wobei der Antikörper nicht spezifisch an das Plasmaprotein bindet, das in einem Individuum, das nicht an CKD leidet, nicht posttranslational modifiziert ist.

7. Die *in vitro* Verwendung eines Antikörpers gemäß Anspruch 6, wobei das Protein humanes Serumalbumin ist.

8. Die *in vitro* Verwendung eines Antikörpers gemäß Anspruch 6 oder 7, wobei die posttranslationale Modifikation Guanidylierung ist.

9. Das Verfahren gemäß einem beliebigen der Ansprüche 1 bis 5, wobei der Unterschied im posttranslationalen Modifikationsmuster des Plasmaproteins des zu diagnostizierenden Individuums unter Verwendung des Antikörpers gemäß einem beliebigen der Ansprüche 6 bis 8 detektiert wird.

**Revendications**

1. Procédé de diagnostic et/ou de surveillance d'une insuffisance rénale chronique (IRC) au stade précoce chez un individu, le procédé comprenant les étapes consistant à :

(a) analyser *in vitro* le schéma de modifications post-traductionnelles d'une protéine plasmatique d'un individu à diagnostiquer, la protéine plasmatique étant choisie parmi le groupe constitué d'albumine, bêta-2 microglobuline (β2MG), cystatine C, transferrine, et protéine de liaison au rétinol ; dans lequel la modification post-traductionnelle est choisie parmi le groupe constitué d'une guanidylation d'un ou plusieurs résidus de lysine d'une sérum-albumine humaine choisis parmi les positions K36, K44, K183, K186, K198, K205, K219, K300, K375, K383, K463, K468, K499, K548, K560, K562, K565, K569, K581, K584, K588, K597 et/ou K598 comme décrit dans la SEQ ID N° 1, d'une glycosylation 2xHex, c'est-à-dire l'ajout d'une molécule de glucose à un ou plusieurs résidus de lysine d'une sérum-albumine humaine choisis parmi les positions K28, K375, K460, K499, K548, K565 comme décrit dans la SEQ ID N° 1, et l'ajout ultérieur d'une autre molécule de glucose à la première molécule de glucose ; d'une formylation d'un ou plusieurs résidus de lysine d'une sérum-albumine humaine choisis parmi K36, K214, K219, K223, K375, K437, K449, K558, K560, K562, K565, K569, K588, K598 comme décrit dans la SEQ ID N° 1 ; d'une oxydation de méthionine 119 de bêta-2 microglobuline comme décrit dans la SEQ ID N° 3 ; d'une formylation de l'extrémité N-terminale de bêta-2 microglobuline, d'une carbamylation d'un résidu de lysine ou de l'extrémité N-terminale de bêta-2 microglobuline ; d'une formylation de l'extrémité N-terminale de cystatine C, d'une carbamylation de l'extrémité N-terminale ou de lysine de cystatine C ; d'une guanidylation d'une lysine de transferrine ; et d'une formylation de l'extrémité N-terminale de protéine de liaison au rétinol ;
(b) comparer le schéma de modifications post-traductionnelles de la protéine plasmatique de l'individu à diagnostiquer avec le schéma de modifications post-traductionnelles de la protéine plasmatique d'un individu ne souffrant pas d'IRC ;
(c) détecter une différence dans le schéma de modifications post-traductionnelles de la protéine plasmatique de l'individu à diagnostiquer par rapport au schéma de modifications post-traductionnelles de la protéine plasmatique chez l'individu ne souffrant pas d'IRC ; et
(d) diagnostiquer l'IRC, si la protéine plasmatique de l'individu à diagnostiquer présente au moins une modification post-traductionnelle selon a) ci-dessus qui est absente de la protéine plasmatique de l'individu ne souffrant pas d'IRC.

2. Procédé selon la revendication 1, dans lequel l'individu à diagnostiquer ne présente pas un taux élevé de créatinine et/ou d'albuminurée, de préférence l'individu à diagnostiquer présente un débit de filtration glomérulaire (DFG) supérieur à 90 mL/min.

3. Procédé selon l'une quelconque des revendications 1 ou 2, dans lequel la protéine plasmatique a une séquence d'acides aminés de l'une quelconque des SEQ ID N° 1, 2, 3, 4, 5 ou 6.

4. Procédé selon l'une quelconque des revendications 1 à 3, dans lequel la protéine plasmatique est une sérum-albumine humaine, de préférence une sérum-albumine humaine ayant la SEQ ID NO° 1.

5. Procédé selon l'une quelconque des revendications 1 à 4, dans lequel la détection de la différence de schéma de modifications post-traductionnelles de la protéine plasmatique est effectuée par une analyse de spectre de masse de la protéine plasmatique isolée, ou essai ELISA, Transfert Western, électrophorèse page 2D après détection colorimétrique de la modification post-traductionnelle.

6. Utilisation in vitro d'un anticorps dans un procédé selon l'une quelconque des revendications 1 à 5, dans laquelle l'anticorps se lie spécifiquement à une protéine plasmatique modifiée de manière post-traductionnelle sélectionnée parmi le groupe constitué d'albumine, bêta-2 microglobuline (β2MG), cystatine C, transferrine, et protéine de liaison au rétinol, la protéine plasmatique ayant de préférence une séquence d'acides aminés de l'une quelconque des SEQ ID N° 1, 2, 3, 4, 5 ou 6 ; dans laquelle la modification post-traductionnelle est choisie parmi le groupe constitué d'une guanidylation d'un ou plusieurs résidus de lysine d'une sérum-albumine humaine choisis parmi les positions K36, K44, K183, K186, K198, K205, K219, K300, K375, K383, K463, K468, K499, K548, K560, K562, K565, K569, K581, K584, K588, K597 et/ou K598 comme décrit dans la SEQ ID N° 1, d'une glycosylation 2xHex d'un ou plusieurs résidus de lysine d'une sérum-albumine humaine choisis parmi les positions K28, K375, K460, K499, K548, K565 comme décrit dans la SEQ ID N° 1 ; d'une formylation d'un ou plusieurs résidus de lysine d'une sérum-albumine humaine choisis parmi K36, K214, K219, K223, K375, K437, K449, K558, K560, K562, K565, K569, K588, K598 comme décrit dans la SEQ ID N° 1 ; d'une oxydation de méthionine 119 de bêta-2 microglobuline comme décrit dans la SEQ ID N° 3 ; d'une formylation de l'extrémité N-terminale de bêta-2 microglobuline, d'une carbamylation d'un résidu de lysine ou de l'extrémité N-terminale de bêta-2 microglobuline ; d'une formylation de l'extrémité N-terminale de cystatine C, d'une carbamylation de l'extrémité N-terminale ou de lysine de cystatine C ; d'une guanidylation d'une lysine de transferrine ; et d'une formylation de l'extrémité N-terminale de protéine de liaison au rétinol ; dans laquelle l'anticorps ne se lie pas spécifiquement à la protéine plasmatique qui n'est pas modifiée de manière post-traductionnelle chez un individu ne souffrant pas d'IRC.

7. Utilisation in vitro d'un anticorps selon la revendication 6, dans laquelle la protéine plasmatique est une sérum-albumine humaine.

8. Utilisation in vitro d'un anticorps selon la revendication 6 ou 7, dans lequel la modification post-traductionnelle est une guanidylation.

9. Procédé selon l'une quelconque des revendications 1 à 5, dans lequel la différence dans le schéma de modifications post-traductionnelles de la protéine plasmatique de l'individu à diagnostiquer est détectée en utilisant l'anticorps utilisé selon l'une quelconque des revendications 6 à 8.

Figure 1 A

Figure 1 B

Figure 1 C

Figure 1 D

Figure 1 E

Figure 1

2A

```
  1  MKWVTFISLL  FLFSSAYSRG  VFRRDAHKSE  VAHRF DLGE  ENFKALVLIA
 51  FAQYLQQCPF  EDHVKLVNEV  TEFAKTCVAD  ESAENCDKSL  HTLFGDKLCT
101  VATLRETYGE  MADCCAKQEP  ERNECFLQHK  DDNPNLPRLV  RPEVDVMCTA
151  FHDNEETFLK  KYLYEIARRH  PYFYAPELLF  FA RYKAAFT  ECCQAAD AA
201  CLLPKLDELR  DEGKASSAKQ  RLKCASLQKF  GERAFKAWAV  ARLSQRFPKA
251  EFAEVSKLVT  DLTKVHTECC  HGDLLECADD  RADLAKYICE  NQDSISSKLK
301  ECCEKPLLEK  SHCIAEVEND  EMPADLPSLA  ADFVESKDVC  KNYAEAKDVF
351  LGMFLYEYAR  RHPDYSVVLL  LRLAKTYETT  LEKCCAAADP  HECYAKVFDE
401  FKPLVEEPQN  LIKQNCELFE  QLGEYKFQNA  LLVRYTKKVP  QVSTPTLVEV
451  SRNLGKVGSK  CCKHPEAKRM  PCAEDYLSVV  LNQLCVLHEK  TPVSDRVTKC
501  CTESLVNRRP  CFSALEVDET  YVPKEFNAET  FTFHADICTL  SEKERQIKKQ
551  TALVELVKHK  PKATKEQL A  VMDDFAAFVE   CCKADDKET  CFAEEGKKLV
601  AASQAALGL
```

2B

2C

2D

2E

2F

Figure 2

## Figure 3

FIGURE 4 A

FIGURE 4 B

FIGURE 4 C

FIGURE 4 D

# Figure 5

```
guanidinylated Lysine residues:


  1 MKWVTFISLL FLFSSAYSRG VFRRDAHKSE VAHRFKDLGE ENFKALVLIA
 51 FAQYLQQCPF EDHVKLVNEV TEFAKTCVAD ESAENCDKSL HTLFGDKLCT
101 VATLRETYGE MADCCAKQEP ERNECFLQHK DDNPNLPRLV RPEVDVMCTA
151 FHDNEETFLK KYLYEIARRH PYFYAPELLF FAKRYKAAFT ECCQAADKAA
201 CLLPKLDELR DEGKASSAKQ RLKCASLQKF GERAFKAWAV ARLSQRFPKA
251 EFAEVSKLVT DLTKVHTECC HGDLLECADD RADLAKYICE NQDSISSKLK
301 ECCEKPLLEK SHCIAEVEND EMPADLPSLA ADFVESKDVC KNYAEAKDVF
351 LGMFLYEYAR RHPDYSVVLL LRLAKTYETT LEKCCAAADP HECYAKVFDE
401 FKPLVEEPQN LIKQNCELFE QLGEYKFQNA LLVRYTKKVP QVSTPTLVEV
451 SRNLGKVGSK CCKHPEAKRM PCAEDYLSVV LNQLCVLHEK TPVSDRVTKC
501 CTESLVNRRP CFSALEVDET YVPKEFNAET FTFHADICTL SEKERQIKKQ
551 TALVELVKHK PKATKEQLKA VMDDFAAFVE KCCKADDKET CFAEEGKKLV
601 AASQAALGL
```

Figure 6

**2-fold Hex glycosylated Lysine residues:**

```
  1 MKWVTFISLL FLFSSAYSRG VFRRDAHKSE VAHRFKDLGE ENFKALVLIA
 51 FAQYLQQCPF EDHVKLVNEV TEFAKTCVAD ESAENCDKSL HTLFGDKLCT
101 VATLRETYGE MADCCAKQEP ERNECFLQHK DDNPNLPRLV RPEVDVMCTA
151 FHDNEETFLK KYLYEIARRH PYFYAPELLF FAKRYKAAFT ECCQAADKAA
201 CLLPKLDELR DEGKASSAKQ RLKCASLQKF GERAFKAWAV ARLSQRFPKA
251 EFAEVSKLVT DLTKVHTECC HGDLLECADD RADLAKYICE NQDSISSKLK
301 ECCEKPLLEK SHCIAEVEND EMPADLPSLA ADFVESKDVC KNYAEAKDVF
351 LGMFLYEYAR RHPDYSVVLL LRLAKTYETT LEKCCAAADP HECYAKVFDE
401 FKPLVEEPQN LIKQNCELFE QLGEYKFQNA LLVRYTKKVP QVSTPTLVEV
451 SRNLGKVGSK CCKHPEAKRM PCAEDYLSVV LNQLCVLHEK TPVSDRVTKC
501 CTESLVNRRP CFSALEVDET YVPKEFNAET FTFHADICTL SEKERQIKKQ
551 TALVELVKHK PKATKEQLKA VMDDFAAFVE KCCKADDKET CFAEEGKKLV
601 AASQAALGL
```

EP 3 132 269 B1

## Figure 7

**Formylated Lysine residues:**

```
  1 MKWVTFISLL FLFSSAYSRG VFRRDAHKSE VAHRFKDLGE ENFKALVLIA
 51 FAQYLQQCPF EDHVKLVNEV TEFAKTCVAD ESAENCDKSL HTLFGDKLCT
101 VATLRETYGE MADCCAKQEP ERNECFLQHK DDNPNLPRLV RPEVDVMCTA
151 FHDNEETFLK KYLYEIARRH PYFYAPELLF FAKRYKAAFT ECCQAADKAA
201 CLLPKLDELR DEGKASSAKQ RLKCASLQKF GERAFKAWAV ARLSQRFPKA
251 EFAEVSKLVT DLTKVHTECC HGDLLECADD RADLAKYICE NQDSISSKLK
301 ECCEKPLLEK SHCIAEVEND EMPADLPSLA ADFVESKDVC KNYAEAKDVF
351 LGMFLYEYAR RHPDYSVVLL LRLAKTYETT LEKCCAAADP HECYAKVFDE
401 FKPLVEEPQN LIKQNCELFE QLGEYKFQNA LLVRYTKKVP QVSTPTLVEV
451 SRNLGKVGSK CCKHPEAKRM PCAEDYLSVV LNQLCVLHEK TPVSDRVTKC
501 CTESLVNRRP CFSALEVDET YVPKEFNAET FTFHADICTL SEKERQIKKQ
551 TALVELVKHK PKATKEQLKA VMDDFAAFVE KCCKADDKET CFAEEGKKLV
601 AASQAALGL
```

EP 3 132 269 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4816567 A

- EP 14165189

### Non-patent literature cited in the description

- PLANTINGA LC. Socio-economic impact in CKD. *Nephrol Ther.,* 2013
- HALLAN S.I. et al. *JAMA.,* 2012, vol. 308, 2349-2360
- SUN Y.M. et al. *Biochem Biophys Res Commun.,* 2013, vol. 433, 359-361
- PRAKASH M et al. *Indian J Nephrol.,* 2010, vol. 20, 9-14
- ZAGER R.A et al. *Am J Physiol Renal Physiol.,* 2006, vol. 291, F546-556
- WANG. *Nature Medicine,* 2007, vol. 13, 1176-84
- GAIKWAD A.B. et al. *Am J Pathol.,* 2010, vol. 176, 1079-1083
- HAN et al. *Am. J. Kidney. Dis.,* 1997, vol. 30, 36-40
- KAIRAITIS et al. *Nephrol. Dial. Transplant,* 2000, vol. 15, 1431-7
- APOSTOLOV et al. *JASN,* 2010, vol. 21, 1852-7
- APOSTOLOV et al. *J. Renal. Nutr.,* 2012, vol. 22, 134-8
- APOSTOLOV, E.O. *Clinical Chemistry,* 2005, vol. 51, 719-28
- APOSTOLOV, E.O. et al. *J. Renal Nutr.,* 2012, vol. 22, 134-8
- LUIZ CARLOS CICHOTA et al. *Journal of Clinical Laboratory Analysis,* 2008, vol. 22, 1-5
- CARLO DONADIO et al. *Clinica Chimica Acta,* 2012, vol. 413, 391-395
- AGARWAL, R. *Am. J. Physiol.,* 2005, vol. 288, F265-71
- CHEN HUANG-HAN et al. *Analyt. Chem.,* 2012, vol. 84, 8635-41
- HIGASHIMOTO Y. et al. *JBC,* 2000, vol. 275, 23199-23203
- SMITH W.G. et al. *Clin. Chim. Acta,* 1988, vol. 178, 297-303
- SHAKLAI N. et al. *J. Biol. Chem.,* 1984, vol. 259, 3812-3817
- HANSEN, H.P. et al. *Kidney International,* 2002, vol. 61, 163-168
- CARTER et al. *Adv Protein Chem.,* 1994, vol. 45, 153-203

- DEEB et al. *Biopolymers.,* 2011, vol. 93, 161-170
- FANALI et al. *Mol Aspects Med.,* 2012, vol. 33, 209-290
- MAHMOODI et al. *Lancet,* 2012, vol. 380, 1649-1661
- WADA et al. *Clin Exp Nephrol.,* 2012, vol. 16, 96-101
- LIM et al. *Nephrology (Carlton).,* 2007, vol. 12, 18-24
- GEKLE et al. *J Am Soc Nephrol.,* 1998, vol. 9, 960-968
- MACCONI et al. *J Am Soc Nephrol.,* 2009, vol. 20, 123-130
- CESSAC et al. *Mech Ageing Dev.,* 1993, vol. 70, 139-148
- WATANABE et al. *Drug Metab Dispos.,* 2012, vol. 40, 1423-1428
- KLAMMT et al. *Nephrol Dial Transplant.,* 2012, vol. 27, 2377-2383
- LORENZO SELLARES et al. *Nefrologia,* 2008, vol. 28 (3), 67-78
- OETTL ; STAUBER. *Br J Pharmacol.,* 2007, vol. 151, 580-590
- LEVEY et al. *Kidney International,* 2011, vol. 80, 17-28
- KDIGO 2012 Clinical Practice Guideline for the Evaluation and Management of Chronic Kidney Disease. *Kidney Int,* 2013, vol. 3, 1-150
- STEVENS et al. *N Engl J Med.,* 08 June 2006, vol. 354 (23), 2473-83
- JONG ; CURHAN. *J Am Soc Nephrol,* 2006, vol. 17, 2120-2126
- KRAGH-HANSEN et al. *Eur J Biochem.,* 05 October 1990, vol. 193 (1), 169-74
- ROZMAN. *J Mass Spectrom.,* 2011, vol. 46, 949-955
- KOHLER et al. *Nature,* 1975, vol. 256, 495
- CLACKSON et al. *Nature,* 1991, vol. 352, 624-628
- MARKS et al. *J. Mol. Biol.,* 1991, vol. 222, 581-597

- Methods in Molecular Biology. **JOHN R. CROWTHER.** The ELISA Guidebook. Humana Press, 2000, vol. 149
- **IRIKURA et al.** *Chem Pharm Bull (Tokyo),* 1991, vol. 39, 724-728
- **KRAGH-HANSEN.** *1 Biochem J.,* 1991, vol. 273, 641-644
- **SARNATSKAYA et al.** *Biochem Pharmacol.,* 2002, vol. 63, 1287-1296
- **LAEMMLI.** *Nature,* 1970, vol. 227, 680-685
- **KIMMEL.** *Meth. Enzymology.,* 1967, 584

SEQUENCE LISTING

<110> eXcorLab GmbH

<120> Diagnosis of Chronic Kidney Disease by Quantitative Analysis of Post-Translational Modifications of Plasma Proteins

<130> PCT89701HV163pau

<140> not yet assigned
<141> 2014-04-17

<150> EP14165189.3
<151> 2014-04-17

<160> 6

<170> BiSSAP 1.3

<210> 1
<211> 609
<212> PRT
<213> Homo sapiens

<220>
<223> P02768 HUMAN Serum albumin Isoform 1

<400> 1

```
Met Lys Trp Val Thr Phe Ile Ser Leu Leu Phe Leu Phe Ser Ser Ala
1               5                   10                  15
Tyr Ser Arg Gly Val Phe Arg Arg Asp Ala His Lys Ser Glu Val Ala
            20                  25                  30
His Arg Phe Lys Asp Leu Gly Glu Glu Asn Phe Lys Ala Leu Val Leu
        35                  40                  45
Ile Ala Phe Ala Gln Tyr Leu Gln Gln Cys Pro Phe Glu Asp His Val
    50                  55                  60
Lys Leu Val Asn Glu Val Thr Glu Phe Ala Lys Thr Cys Val Ala Asp
65                  70                  75                  80
Glu Ser Ala Glu Asn Cys Asp Lys Ser Leu His Thr Leu Phe Gly Asp
                85                  90                  95
Lys Leu Cys Thr Val Ala Thr Leu Arg Glu Thr Tyr Gly Glu Met Ala
            100                 105                 110
Asp Cys Cys Ala Lys Gln Glu Pro Glu Arg Asn Glu Cys Phe Leu Gln
            115                 120                 125
His Lys Asp Asp Asn Pro Asn Leu Pro Arg Leu Val Arg Pro Glu Val
        130                 135                 140
Asp Val Met Cys Thr Ala Phe His Asp Asn Glu Glu Thr Phe Leu Lys
145                 150                 155                 160
Lys Tyr Leu Tyr Glu Ile Ala Arg Arg His Pro Tyr Phe Tyr Ala Pro
                165                 170                 175
Glu Leu Leu Phe Phe Ala Lys Arg Tyr Lys Ala Ala Phe Thr Glu Cys
            180                 185                 190
Cys Gln Ala Ala Asp Lys Ala Ala Cys Leu Leu Pro Lys Leu Asp Glu
        195                 200                 205
Leu Arg Asp Glu Gly Lys Ala Ser Ser Ala Lys Gln Arg Leu Lys Cys
        210                 215                 220
Ala Ser Leu Gln Lys Phe Gly Glu Arg Ala Phe Lys Ala Trp Ala Val
225                 230                 235                 240
Ala Arg Leu Ser Gln Arg Phe Pro Lys Ala Glu Phe Ala Glu Val Ser
                245                 250                 255
Lys Leu Val Thr Asp Leu Thr Lys Val His Thr Glu Cys Cys His Gly
            260                 265                 270
Asp Leu Leu Glu Cys Ala Asp Asp Arg Ala Asp Leu Ala Lys Tyr Ile
```

```
                    275                     280                     285
        Cys Glu Asn Gln Asp Ser Ile Ser Ser Lys Leu Lys Glu Cys Cys Glu
            290                     295                     300
        Lys Pro Leu Leu Glu Lys Ser His Cys Ile Ala Glu Val Glu Asn Asp
        305                     310                     315                     320
        Glu Met Pro Ala Asp Leu Pro Ser Leu Ala Ala Asp Phe Val Glu Ser
                            325                     330                     335
        Lys Asp Val Cys Lys Asn Tyr Ala Glu Ala Lys Asp Val Phe Leu Gly
                    340                     345                     350
        Met Phe Leu Tyr Glu Tyr Ala Arg Arg His Pro Asp Tyr Ser Val Val
                    355                     360                     365
        Leu Leu Leu Arg Leu Ala Lys Thr Tyr Glu Thr Thr Leu Glu Lys Cys
            370                     375                     380
        Cys Ala Ala Ala Asp Pro His Glu Cys Tyr Ala Lys Val Phe Asp Glu
        385                     390                     395                     400
        Phe Lys Pro Leu Val Glu Glu Pro Gln Asn Leu Ile Lys Gln Asn Cys
                            405                     410                     415
        Glu Leu Phe Glu Gln Leu Gly Glu Tyr Lys Phe Gln Asn Ala Leu Leu
                    420                     425                     430
        Val Arg Tyr Thr Lys Lys Val Pro Gln Val Ser Thr Pro Thr Leu Val
                    435                     440                     445
        Glu Val Ser Arg Asn Leu Gly Lys Val Gly Ser Lys Cys Cys Lys His
            450                     455                     460
        Pro Glu Ala Lys Arg Met Pro Cys Ala Glu Asp Tyr Leu Ser Val Val
        465                     470                     475                     480
        Leu Asn Gln Leu Cys Val Leu His Glu Lys Thr Pro Val Ser Asp Arg
                    485                     490                     495
        Val Thr Lys Cys Cys Thr Glu Ser Leu Val Asn Arg Arg Pro Cys Phe
                    500                     505                     510
        Ser Ala Leu Glu Val Asp Glu Thr Tyr Val Pro Lys Glu Phe Asn Ala
                    515                     520                     525
        Glu Thr Phe Thr Phe His Ala Asp Ile Cys Thr Leu Ser Glu Lys Glu
            530                     535                     540
        Arg Gln Ile Lys Lys Gln Thr Ala Leu Val Glu Leu Val Lys His Lys
        545                     550                     555                     560
        Pro Lys Ala Thr Lys Glu Gln Leu Lys Ala Val Met Asp Asp Phe Ala
                            565                     570                     575
        Ala Phe Val Glu Lys Cys Cys Lys Ala Asp Asp Lys Glu Thr Cys Phe
                    580                     585                     590
        Ala Glu Glu Gly Lys Lys Leu Val Ala Ala Ser Gln Ala Ala Leu Gly
            595                     600                     605
        Leu
```

<210> 2

<211> 417

<212> PRT

<213> Homo sapiens

<220>

<223> P02768 HUMAN Serum albumin Isoform 2

<400> 2

```
Met Lys Trp Val Thr Phe Ile Ser Leu Leu Phe Leu Phe Ser Ser Ala
1               5                   10              15
Tyr Ser Arg Gly Val Phe Arg Arg Asp Ala His Lys Ser Glu Val Ala
            20              25              30
His Arg Phe Lys Asp Leu Gly Glu Glu Asn Phe Lys Ala Trp Ala Val
        35              40              45
Ala Arg Leu Ser Gln Arg Phe Pro Lys Ala Glu Phe Ala Glu Val Ser
        50              55              60
Lys Leu Val Thr Asp Leu Thr Lys Val His Thr Glu Cys Cys His Gly
65                  70              75              80


Asp Leu Leu Glu Cys Ala Asp Asp Arg Ala Asp Leu Ala Lys Tyr Ile
                85              90              95
Cys Glu Asn Gln Asp Ser Ile Ser Ser Lys Leu Lys Glu Cys Cys Glu
            100             105             110
Lys Pro Leu Leu Glu Lys Ser His Cys Ile Ala Glu Val Glu Asn Asp
        115             120             125
Glu Met Pro Ala Asp Leu Pro Ser Leu Ala Ala Asp Phe Val Glu Ser
    130             135             140
Lys Asp Val Cys Lys Asn Tyr Ala Glu Ala Lys Asp Val Phe Leu Gly
145             150             155             160
Met Phe Leu Tyr Glu Tyr Ala Arg Arg His Pro Asp Tyr Ser Val Val
            165             170             175
Leu Leu Leu Arg Leu Ala Lys Thr Tyr Glu Thr Thr Leu Glu Lys Cys
            180             185             190
Cys Ala Ala Ala Asp Pro His Glu Cys Tyr Ala Lys Val Phe Asp Glu
            195             200             205
Phe Lys Pro Leu Val Glu Glu Pro Gln Asn Leu Ile Lys Gln Asn Cys
    210             215             220
Glu Leu Phe Glu Gln Leu Gly Glu Tyr Lys Phe Gln Asn Ala Leu Leu
225             230             235             240
Val Arg Tyr Thr Lys Lys Val Pro Gln Val Ser Thr Pro Thr Leu Val
            245             250             255
Glu Val Ser Arg Asn Leu Gly Lys Val Gly Ser Lys Cys Cys Lys His
            260             265             270
Pro Glu Ala Lys Arg Met Pro Cys Ala Glu Asp Tyr Leu Ser Val Val
    275             280             285
Leu Asn Gln Leu Cys Val Leu His Glu Lys Thr Pro Val Ser Asp Arg
    290             295             300
Val Thr Lys Cys Cys Thr Glu Ser Leu Val Asn Arg Arg Pro Cys Phe
305             310             315             320
Ser Ala Leu Glu Val Asp Glu Thr Tyr Val Pro Lys Glu Phe Asn Ala
            325             330             335
Glu Thr Phe Thr Phe His Ala Asp Ile Cys Thr Leu Ser Glu Lys Glu
            340             345             350
Arg Gln Ile Lys Lys Gln Thr Ala Leu Val Glu Leu Val Lys His Lys
            355             360             365
Pro Lys Ala Thr Lys Glu Gln Leu Lys Ala Val Met Asp Asp Phe Ala
    370             375             380
Ala Phe Val Glu Lys Cys Cys Lys Ala Asp Asp Lys Glu Thr Cys Phe
385             390             395             400
Ala Glu Glu Gly Lys Lys Leu Val Ala Ala Ser Gln Ala Ala Leu Gly
            405             410             415
Leu
```

<210> 3

<211> 119

<212> PRT

<213> Homo sapiens

24

<220>
<223> P61769 B2MG_HUMAN Beta-2-microglobulin

<400> 3

```
Met Ser Arg Ser Val Ala Leu Ala Val Leu Ala Leu Leu Ser Leu Ser
1               5                   10                  15
Gly Leu Glu Ala Ile Gln Arg Thr Pro Lys Ile Gln Val Tyr Ser Arg
            20                  25                  30
His Pro Ala Glu Asn Gly Lys Ser Asn Phe Leu Asn Cys Tyr Val Ser
            35                  40                  45
Gly Phe His Pro Ser Asp Ile Glu Val Asp Leu Leu Lys Asn Gly Glu
        50                  55                  60
Arg Ile Glu Lys Val Glu His Ser Asp Leu Ser Phe Ser Lys Asp Trp
65                  70                  75                  80
Ser Phe Tyr Leu Leu Tyr Tyr Thr Glu Phe Thr Pro Thr Glu Lys Asp
                85                  90                  95
Glu Tyr Ala Cys Arg Val Asn His Val Thr Leu Ser Gln Pro Lys Ile
            100                 105                 110
Val Lys Trp Asp Arg Asp Met
            115
```

<210> 4
<211> 146
<212> PRT
<213> Homo sapiens

<220>
<223> P01034 CYTC_HUMAN Cystatin-C

<400> 4

```
Met Ala Gly Pro Leu Arg Ala Pro Leu Leu Leu Leu Ala Ile Leu Ala
1               5                   10                  15
Val Ala Leu Ala Val Ser Pro Ala Ala Gly Ser Ser Pro Gly Lys Pro
            20                  25                  30
Pro Arg Leu Val Gly Gly Pro Met Asp Ala Ser Val Glu Glu Glu Gly
            35                  40                  45
Val Arg Arg Ala Leu Asp Phe Ala Val Gly Glu Tyr Asn Lys Ala Ser
        50                  55                  60
Asn Asp Met Tyr His Ser Arg Ala Leu Gln Val Val Arg Ala Arg Lys
65                  70                  75                  80
Gln Ile Val Ala Gly Val Asn Tyr Phe Leu Asp Val Glu Leu Gly Arg
                85                  90                  95
Thr Thr Cys Thr Lys Thr Gln Pro Asn Leu Asp Asn Cys Pro Phe His
            100                 105                 110
Asp Gln Pro His Leu Lys Arg Lys Ala Phe Cys Ser Phe Gln Ile Tyr
            115                 120                 125
Ala Val Pro Trp Gln Gly Thr Met Thr Leu Ser Lys Ser Thr Cys Gln
            130                 135                 140
Asp Ala
145
```

<210> 5
<211> 698
<212> PRT
<213> Homo sapiens

<220>
<223> P02787 TRFE_HUMAN Serotransferrin

<400> 5

```
Met Arg Leu Ala Val Gly Ala Leu Leu Val Cys Ala Val Leu Gly Leu
1               5                   10                  15
Cys Leu Ala Val Pro Asp Lys Thr Val Arg Trp Cys Ala Val Ser Glu
            20                  25                  30
His Glu Ala Thr Lys Cys Gln Ser Phe Arg Asp His Met Lys Ser Val
            35                  40                  45
Ile Pro Ser Asp Gly Pro Ser Val Ala Cys Val Lys Lys Ala Ser Tyr
        50                  55                  60
Leu Asp Cys Ile Arg Ala Ile Ala Ala Asn Glu Ala Asp Ala Val Thr
65                  70                  75                  80
Leu Asp Ala Gly Leu Val Tyr Asp Ala Tyr Leu Ala Pro Asn Asn Leu
                85                  90                  95
Lys Pro Val Val Ala Glu Phe Tyr Gly Ser Lys Glu Asp Pro Gln Thr
            100                 105                 110
Phe Tyr Tyr Ala Val Ala Val Val Lys Lys Asp Ser Gly Phe Gln Met
            115                 120                 125
```

```
Asn Gln Leu Arg Gly Lys Lys Ser Cys His Thr Gly Leu Gly Arg Ser
    130                 135                 140
Ala Gly Trp Asn Ile Pro Ile Gly Leu Leu Tyr Cys Asp Leu Pro Glu
145                 150                 155                 160
Pro Arg Lys Pro Leu Glu Lys Ala Val Ala Asn Phe Phe Ser Gly Ser
            165                 170                 175
Cys Ala Pro Cys Ala Asp Gly Thr Asp Phe Pro Gln Leu Cys Gln Leu
        180                 185                 190
Cys Pro Gly Cys Gly Cys Ser Thr Leu Asn Gln Tyr Phe Gly Tyr Ser
        195                 200                 205
Gly Ala Phe Lys Cys Leu Lys Asp Gly Ala Gly Asp Val Ala Phe Val
    210                 215                 220
Lys His Ser Thr Ile Phe Glu Asn Leu Ala Asn Lys Ala Asp Arg Asp
225                 230                 235                 240
Gln Tyr Glu Leu Leu Cys Leu Asp Asn Thr Arg Lys Pro Val Asp Glu
            245                 250                 255
Tyr Lys Asp Cys His Leu Ala Gln Val Pro Ser His Thr Val Val Ala
        260                 265                 270
Arg Ser Met Gly Gly Lys Glu Asp Leu Ile Trp Glu Leu Leu Asn Gln
    275                 280                 285
Ala Gln Glu His Phe Gly Lys Asp Lys Ser Lys Glu Phe Gln Leu Phe
    290                 295                 300
Ser Ser Pro His Gly Lys Asp Leu Leu Phe Lys Asp Ser Ala His Gly
305                 310                 315                 320
Phe Leu Lys Val Pro Pro Arg Met Asp Ala Lys Met Tyr Leu Gly Tyr
            325                 330                 335
Glu Tyr Val Thr Ala Ile Arg Asn Leu Arg Glu Gly Thr Cys Pro Glu
        340                 345                 350
Ala Pro Thr Asp Glu Cys Lys Pro Val Lys Trp Cys Ala Leu Ser His
        355                 360                 365
His Glu Arg Leu Lys Cys Asp Glu Trp Ser Val Asn Ser Val Gly Lys
    370                 375                 380
Ile Glu Cys Val Ser Ala Glu Thr Thr Glu Asp Cys Ile Ala Lys Ile
385                 390                 395                 400
Met Asn Gly Glu Ala Asp Ala Met Ser Leu Asp Gly Gly Phe Val Tyr
                405                 410                 415
Ile Ala Gly Lys Cys Gly Leu Val Pro Val Leu Ala Glu Asn Tyr Asn
            420                 425                 430
Lys Ser Asp Asn Cys Glu Asp Thr Pro Glu Ala Gly Tyr Phe Ala Ile
        435                 440                 445
Ala Val Val Lys Lys Ser Ala Ser Asp Leu Thr Trp Asp Asn Leu Lys
    450                 455                 460
Gly Lys Lys Ser Cys His Thr Ala Val Gly Arg Thr Ala Gly Trp Asn
465                 470                 475                 480
Ile Pro Met Gly Leu Leu Tyr Asn Lys Ile Asn His Cys Arg Phe Asp
            485                 490                 495
Glu Phe Phe Ser Glu Gly Cys Ala Pro Gly Ser Lys Lys Asp Ser Ser
        500                 505                 510
Leu Cys Lys Leu Cys Met Gly Ser Gly Leu Asn Leu Cys Glu Pro Asn
        515                 520                 525
Asn Lys Glu Gly Tyr Tyr Gly Tyr Thr Gly Ala Phe Arg Cys Leu Val
    530                 535                 540
Glu Lys Gly Asp Val Ala Phe Val Lys His Gln Thr Val Pro Gln Asn
545                 550                 555                 560
Thr Gly Gly Lys Asn Pro Asp Pro Trp Ala Lys Asn Leu Asn Glu Lys
                565                 570                 575
Asp Tyr Glu Leu Leu Cys Leu Asp Gly Thr Arg Lys Pro Val Glu Glu
        580                 585                 590
Tyr Ala Asn Cys His Leu Ala Arg Ala Pro Asn His Ala Val Val Thr
        595                 600                 605
Arg Lys Asp Lys Glu Ala Cys Val His Lys Ile Leu Arg Gln Gln Gln
    610                 615                 620
His Leu Phe Gly Ser Asn Val Thr Asp Cys Ser Gly Asn Phe Cys Leu
```

```
          625                       630                       635                       640
Phe Arg Ser Glu Thr Lys Asp Leu Leu Phe Arg Asp Asp Thr Val Cys
                  645                       650                       655
Leu Ala Lys Leu His Asp Arg Asn Thr Tyr Glu Lys Tyr Leu Gly Glu
                  660                       665                       670
Glu Tyr Val Lys Ala Val Gly Asn Leu Arg Lys Cys Ser Thr Ser Ser
                  675                       680                       685
Leu Leu Glu Ala Cys Thr Phe Arg Arg Pro
                  690                       695
```

<210> 6
<211> 201
<212> PRT
<213> Homo sapiens

<220>
<223> P02753 RET4_HUMAN Retinol-binding protein 4

<400> 6

```
Met Lys Trp Val Trp Ala Leu Leu Leu Leu Ala Ala Leu Gly Ser Gly
1               5                   10                  15
Arg Ala Glu Arg Asp Cys Arg Val Ser Ser Phe Arg Val Lys Glu Asn
            20                  25                  30
Phe Asp Lys Ala Arg Phe Ser Gly Thr Trp Tyr Ala Met Ala Lys Lys
            35                  40                  45
Asp Pro Glu Gly Leu Phe Leu Gln Asp Asn Ile Val Ala Glu Phe Ser
        50                  55                  60
Val Asp Glu Thr Gly Gln Met Ser Ala Thr Ala Lys Gly Arg Val Arg
65                  70                  75                  80
Leu Leu Asn Asn Trp Asp Val Cys Ala Asp Met Val Gly Thr Phe Thr
                85                  90                  95
Asp Thr Glu Asp Pro Ala Lys Phe Lys Met Lys Tyr Trp Gly Val Ala
            100                 105                 110
Ser Phe Leu Gln Lys Gly Asn Asp Asp His Trp Ile Val Asp Thr Asp
            115                 120                 125
Tyr Asp Thr Tyr Ala Val Gln Tyr Ser Cys Arg Leu Leu Asn Leu Asp
        130                 135                 140
Gly Thr Cys Ala Asp Ser Tyr Ser Phe Val Phe Ser Arg Asp Pro Asn
145                 150                 155                 160
Gly Leu Pro Pro Glu Ala Gln Lys Ile Val Arg Gln Arg Gln Glu Glu
                165                 170                 175
Leu Cys Leu Ala Arg Gln Tyr Arg Leu Ile Val His Asn Gly Tyr Cys
            180                 185                 190
Asp Gly Arg Ser Glu Arg Asn Leu Leu
        195                 200
```